(19) Europäisches Patentamt
European Patent Office
Office européen des brevets

(11) EP 3 950 058 A1

(12) EUROPEAN PATENT APPLICATION
published in accordance with Art. 153(4) EPC

(43) Date of publication:
**09.02.2022 Bulletin 2022/06**

(21) Application number: **20784332.7**

(22) Date of filing: **02.04.2020**

(51) International Patent Classification (IPC):
***A61P 5/18*** (2006.01) ***A61P 43/00*** (2006.01)
***A61K 9/08*** (2006.01) ***A61K 47/02*** (2006.01)
***A61K 47/10*** (2017.01) ***A61K 47/12*** (2006.01)
***A61K 47/26*** (2006.01) ***A61K 31/198*** (2006.01)

(52) Cooperative Patent Classification (CPC):
**A61K 9/08; A61K 31/198; A61K 47/02; A61K 47/10; A61K 47/12; A61K 47/26; A61P 5/18; A61P 5/20; A61P 43/00**

(86) International application number:
**PCT/JP2020/015129**

(87) International publication number:
**WO 2020/204117 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2019 JP 2019071168**

(71) Applicant: **EA Pharma Co., Ltd.**
**Tokyo 104-0042 (JP)**

(72) Inventors:
- **MAKITA Yoshimichi**
  **Nagoya-shi, Aichi 461-8631 (JP)**
- **KATAOKA Daisuke**
  **Tokyo 164-0011 (JP)**
- **KUYAMA Kazuo**
  **Nagoya-shi, Aichi 461-8631 (JP)**
- **ASANO Kenji**
  **Nagoya-shi, Aichi 461-8631 (JP)**
- **SHIBASAKI Ryotaro**
  **Kawasaki-shi, Kanagawa 210-8681 (JP)**
- **YAMAMOTO Akiko**
  **Kawasaki-shi, Kanagawa 210-8681 (JP)**
- **WAGATSUMA Hirotaka**
  **Kawasaki-shi, Kanagawa 210-8681 (JP)**
- **TSURUTA Atsushi**
  **Kawasaki-shi, Kanagawa 210-8681 (JP)**
- **TAKANOHASHI Toshiyuki**
  **Kawasaki-shi, Kanagawa 210-8681 (JP)**

(74) Representative: **Mewburn Ellis LLP**
**Aurora Building**
**Counterslip**
**Bristol BS1 6BX (GB)**

# (54) PHARMACEUTICAL COMPOSITION HAVING FAVORABLE STABILITY

(57) Provided is a medicinal composition having excellent stability, the medicinal composition comprising 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition has a pH of 8.0 or less.

[Figure 1]

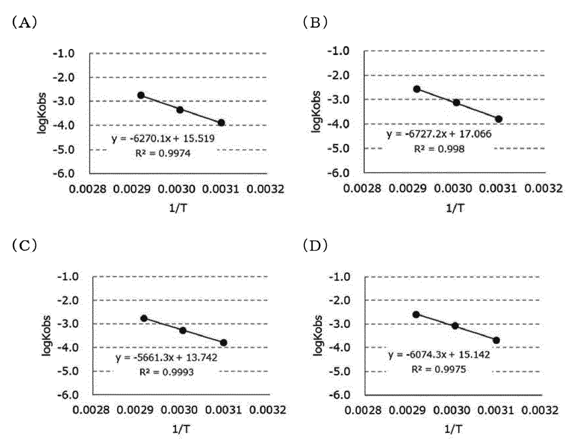


EP 3 950 058 A1

Printed by Jouve, 75001 PARIS (FR)

## Description

FIELD OF THE INVENTION

The present invention relates to a medicinal composition comprising 3-{[(2S)-2-amino-2-carboxyethyl]carbamoylamino}-5-chloro-4-methylbenzenesulfonic acid and the like and having a predetermined pH. The medicinal composition according to the present invention can be used, for example, for treatments of secondary hyperparathyroidism under maintenance dialysis.

BACKGROUND ART

A calcium receptor, also called a calcium sensing receptor (CaSR), was cloned from bovine thyroid in 1993 as G-protein coupled seven-transmembrane receptor (G-protein coupled receptor (GPCR)) that senses extracellular calcium ($Ca^{2+}$). The calcium receptor has a function of changing an intracellular $Ca^{2+}$ concentration by sensing extracellular $Ca^{2+}$ and thus regulating production of hormones and the like involved in $Ca^{2+}$ metabolic regulation, such as parathyroid hormone.

Recently, it has been clarified that cinacalcet (CCT) that is a calcium receptor agonist has an effect of suppressing secretion of parathyroid hormone by acting on the calcium receptor of parathyroid to enhance $Ca^{2+}$ sensitivity of the calcium receptor, and the cinacalcet has been marketed as a therapeutic drug for secondary hyperparathyroidism in dialysis patients.

In addition, it has been clarified that the calcium receptor is expressed even in kidney, brain, thyroid, bones, and gastrointestinal tracts, and thus, it is considered that the calcium receptor is involved in various diseases.

Each of WO 2011/108690 A (Patent document 1) and JP 2013-63971 A (Patent document 2) discloses, as a calcium receptor agonist, an alkylamine derivate comprising (2S)-2-amino-3-{[(3-chloro-2-methyl-5-sulfophenyl)carbamoyl]amino}propanoic acid (another name: 3-{[(2S)-2-amino-2-carboxyethyl]carbamoylamino}-5-chloro-4-methylbenzenesulfonic acid) or a salt thereof. Each of Patent Literatures 1 and 2 discloses a therapeutic agent for hyperparathyroidism (for example, secondary hyperparathyroidism under maintenance dialysis) using the alkylamine derivate and the salt thereof, a therapeutic agent for diarrhea and peptic ulcer, and the like. Furthermore, each of Patent Literatures 1 and 2 also discloses that these therapeutic agents are used for an oral formulation or a parenteral formulation (for example, a formulation for dialysis patients).

PRIOR ART DOCUMENTS Patent documents

Patent document 1: WO 2011/108690 A
Patent document 2: JP 2013-63971 A

SUMMARY OF INVENTION

Problem to be Solved by the Invention

In a pharmaceutical formulation, in particular, a liquid formulation, stability is important in terms of quality control. Therefore, it is desirable to provide a pharmaceutical formulation having excellent stability even in pharmaceuticals comprising 3-{[(2S)-2-amino-2-carboxyethyl]carbamoylamino}-5-chloro-4-methylbenzenesulfonic acid or a salt thereof.

Means for Solving Problem

As a result of intensive studies from the viewpoint of stability, the present invention provides the following medicinal composition, a treatment method, and the like.

(1) A medicinal composition containing, as an active ingredient, 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition has a pH of 8.0 or less.
(2) The medicinal composition according to (1), further containing a pH adjuster and/or a buffer.
(3) The medicinal composition according to (1) or (2), wherein a pH adjuster is one or a combination of two or more selected from the group consisting of an organic acid such as acetic acid, citric acid, succinic acid, and tartaric acid,

and salts thereof; an inorganic acid such as hydrochloric acid and phosphoric acid, and salts thereof; and an inorganic base such as sodium hydroxide and ammonia water.

(4) The medicinal composition according to any one of (1) to (3), wherein a pH adjuster is one or a combination of two or more selected from the group consisting of phosphoric acid and salts thereof.

(5) The medicinal composition according to any one of (1) to (4), wherein a pH adjuster is disodium hydrogen phosphate hydrate or sodium dihydrogen phosphate.

(6) The medicinal composition according to any one of (1) to (5), wherein the medicinal composition has a pH of 5.0 to 7.5.

(7) The medicinal composition according to any one of (1) to (6), wherein the medicinal composition has a pH of 6.0 to 7.0.

(8) The medicinal composition according to any one of (1) to (7), further containing a pharmaceutically acceptable tonicity agent.

(9) The medicinal composition according to (8), wherein the tonicity agent is one or a combination of two or more selected from the group consisting of sodium chloride, D-mannitol, glycerin, concentrated glycerin, glucose, and propylene glycol.

(10) The medicinal composition according to (9), wherein the tonicity agent is sodium chloride.

(11) The medicinal composition according to any one of (1) to (10), wherein the medicinal composition is a liquid formulation.

(12) The medicinal composition according to any one of (1) to (11), wherein the medicinal composition is used for a treatment of secondary hyperparathyroidism under maintenance dialysis.

(13) The medicinal composition according to any one of (1) to (12), wherein the active ingredient is used to be intravenously administered to an adult patient with secondary hyperparathyroidism under maintenance dialysis at the end of dialysis with a daily dose of 0.025 mg to 0.8 mg.

(14) The medicinal composition according to (13), wherein the dose is 0.05 mg to 0.2 mg.

(15) The medicinal composition according to (13) or (14), wherein the end of dialysis is the end of each dialysis session in a dialysis schedule of 3 to 5 sessions a week.

(16) A method for treating secondary hyperparathyroidism under maintenance dialysis using the medicinal composition according to any one of (1) to (15).

(17) A pharmaceutical formulation comprising 3-{[(2S)-2-amino-2-carboxyethyl]carbamoylamino}-5-chloro-4-methylbenzenesulfonic acid, a pharmaceutically acceptable salt thereof, or a solvate thereof, wherein the pharmaceutical formulation is used by filling a vial, a syringe, a bag, or a bottle with a medicinal composition having a pH of 8.0 or less.

(18) A method for stabilizing a medicinal composition comprising 3-{[(2S)-2-amino-2-carboxyethyl]carbamoylamino}-5-chloro-4-methylbenzenesulfonic acid, a pharmaceutically acceptable salt thereof, or a solvate thereof by adjusting a pH of the medicinal composition to 8.0 or less.

The medicinal composition according to any one of (1) to (15), the treatment method according to (16), the pharmaceutical formulation according to (17), and the stabilization method according to (18) can be used in a combination of usages and doses in medicinal compositions or treatment methods according to the following aspects A to D.

Aspect A: A medicinal composition for preventing or treating, or a method for treating secondary hyperparathyroidism under maintenance dialysis, wherein 3-{[(2S)-2-amino-2-carboxyethyl]carbamoylamino}-5-chloro-4-methylbenzenesulfonic acid, a pharmaceutically acceptable salt thereof, or a solvate thereof is used to be intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

Aspect B: A medicinal composition for preventing or treating, or a method for treating secondary hyperparathyroidism under maintenance dialysis, wherein 3-{[(2S)-2-amino-2-carboxyethyl]carbamoylamino}-5-chloro-4-methylbenzenesulfonic acid, a pharmaceutically acceptable salt thereof, or a solvate thereof is used to reduce side effects by being intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg and further preferably 0.05 to 0.2 mg.

Aspect C: A medicinal composition for preventing or treating, or a method for treating secondary hyperparathyroidism under maintenance dialysis, wherein 3-{[(2S)-2-amino-2-carboxyethyl]carbamoylamino}-5-chloro-4-methylbenzenesulfonic acid, a pharmaceutically acceptable salt thereof, or a solvate thereof is used to manifest no significant accumulating property by being intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg and further preferably 0.05 to 0.2 mg.

Aspect D: A medicinal composition for preventing or treating, or a method for treating secondary hyperparathyroidism under maintenance dialysis, wherein 3-{[(2S)-2-amino-2-carboxyethyl]carbamoylamino}-5-chloro-4-methylbenzenesulfonic acid, a pharmaceutically acceptable salt thereof, or a solvate thereof is used for long-term administration by being intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

The medicinal composition or the treatment method according to any one of the aspects A to D, wherein the end of dialysis is the end of each dialysis session in a dialysis schedule of 3 to 5 sessions a week.

The medicinal composition or the treatment method according to any one of the aspects A to D, wherein the 3-{[(2S)-2-amino-2-carboxyethyl]carbamoylamino}-5-chloro-4-methylbenzenesulfonic acid, the pharmaceutically acceptable salt thereof, or the solvate thereof is sodium 3-{[(2S)-2-amino-2-carboxyethyl]carbamoylamino}-5-chloro-4-methylbenzenesulfonate or a solvate thereof.

The medicinal composition or the treatment method according to any one of the aspects A to D, wherein the 3-{[(2S)-2-amino-2-carboxyethyl]carbamoylamino}-5-chloro-4-methylbenzenesulfonic acid, the pharmaceutically acceptable salt thereof, or the solvate thereof is sodium 3-{[(2S)-2-amino-2-carboxyethyl]carbamoylamino}-5-chloro-4-methylbenzenesulfonate.

Preferably, the medicinal composition according to any one of (1) to (15), the treatment method according to (16), the pharmaceutical formulation according to (17), and the stabilization method according to (18) can be used in a combination of usages and doses in medicinal compositions according to the following [1] to [7].

[1] A medicinal composition for preventing or treating secondary hyperparathyroidism under maintenance dialysis, the medicinal composition containing 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition is intravenously administered at the end of dialysis with a daily dose of 0.025 mg to 0.8 mg for adults.

[2] A medicinal composition for preventing or treating secondary hyperparathyroidism under maintenance dialysis with reduced side effects, the medicinal composition containing 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition is intravenously administered at the end of dialysis with a daily dose of 0.025 mg to 0.8 mg for adults.

[3] A medicinal composition for preventing or treating secondary hyperparathyroidism under maintenance dialysis without manifesting significant accumulating property, the medicinal composition containing 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition is intravenously administered at the end of dialysis with a daily dose of 0.025 mg to 0.8 mg for adults.

[4] The medicinal composition according to any one of [1] to [3], wherein the medicinal composition is intravenously administered with a daily dose of 0.025 to 0.4 mg for adults.

[5] The medicinal composition according to any one of [1] to [4], wherein the medicinal composition is intravenously administered at the end of dialysis with a daily dose of 0.05 to 0.2 mg for adults.

[6] The medicinal composition according to any one of [1] to [5], wherein the end of dialysis is the end of each dialysis session in a dialysis schedule of 3 to 5 sessions a week.

[7] The medicinal composition according to any one of [1] to [6], wherein the 3-{[(2S)-2-amino-2-carboxyethyl]carbamoylamino}-5-chloro-4-methylbenzenesulfonic acid, the pharmaceutically acceptable salt thereof, or the solvate thereof is sodium 3-{[(2S)-2-amino-2-carboxyethyl]carbamoylamino}-5-chloro-4-methylbenzenesulfonate or a solvate thereof.

Alternatively, the medicinal composition according to any one of (1) to (15), the treatment method according to (16), the pharmaceutical formulation according to (17), and the stabilization method according to (18) can be used in a combination of usages and doses in medicinal compositions or treatment methods according to the following aspects E to G.

Aspect E: A medicinal composition for preventing or treating, or a method for treating secondary hyperparathyroidism under maintenance dialysis, wherein the medicinal composition comprises, as an active ingredient, 3-{[(2S)-2-amino-2-carboxyethyl]carbamoylamino}-5-chloro-4-methylbenzenesulfonic acid, a pharmaceutically acceptable salt thereof, or a solvate thereof, and the active ingredient is used to regulate a serum PTH concentration to a normal level by being intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

Aspect F: A medicinal composition for preventing or treating, or a method for treating secondary hyperparathyroidism under maintenance dialysis, wherein the medicinal composition comprises, as an active ingredient, 3-{[(2S)-2-amino-2-carboxyethyl]carbamoylamino}-5-chloro-4-methylbenzenesulfonic acid, a pharmaceutically acceptable salt thereof, or a solvate thereof, and the active ingredient is used to regulate serum PTH and Ca concentrations to normal

levels by being intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

Aspect G: A medicinal composition or a method for regulating a serum PTH concentration in a patient with secondary hyperparathyroidism under maintenance dialysis to a normal level, wherein the medicinal composition comprises, as an active ingredient, 3-{[(2S)-2-amino-2-carboxyethyl]carbamoylamino}-5-chloro-4-methylbenzenesulfonic acid, a pharmaceutically acceptable salt thereof, or a solvate thereof, and the active ingredient is intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg. Further, a medicinal composition or a method for regulating serum PTH and Ca concentrations in a patient with secondary hyperparathyroidism under maintenance dialysis to normal levels, wherein 3-{[(2S)-2-amino-2-carboxyethyl]carbamoylamino}-5-chloro-4-methylbenzenesulfonic acid, a pharmaceutically acceptable salt thereof, or a solvate thereof is intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

The present invention also relates to a kit comprising 3-{[(2S)-2-amino-2-carboxyethyl]carbamoylamino}-5-chloro-4-methylbenzenesulfonic acid, a pharmaceutically acceptable salt thereof, or a solvate thereof, and a label and/or an attached instruction instructing a daily dose for intravenous administration to adults at the end of dialysis (for example, a dose of 0.025 mg to 0.8 mg, more preferably 0.025 to 0.4 mg, and further preferably 0.05 to 0.2 mg) for preventing or treating secondary hyperparathyroidism under maintenance dialysis.

The kit may further contain a container for comprising3-{[(2S)-2-amino-2-carboxyethyl]carbamoylamino}-5-chloro-4-methylbenzenesulfonic acid, a pharmaceutically acceptable salt thereof, or a solvate thereof (for example, a vial or an ampoule) and/or a box (package) for packaging the container.

In the kit, the 3-{[(2S)-2-amino-2-carboxyethyl]carbamoylamino}-5-chloro-4-methylbenzenesulfonic acid, the pharmaceutically acceptable salt thereof, or the solvate thereof may be a medicinal composition comprising 3-{[(2S)-2-amino-2-carboxyethyl]carbamoylamino}-5-chloro-4-methylbenzenesulfonic acid, a pharmaceutically acceptable salt thereof, or a solvate thereof, and a pharmaceutically acceptable carrier (for example, sodium chloride, disodium hydrogen phosphate or a hydrate thereof, sodium dihydrogen phosphate or a hydrate thereof).

## Effects of Invention

According to the present invention, it is possible to provide a medicinal composition having excellent stability (for example, long-term storage stability), the medicinal composition comprising 3-{[(2S)-2-amino-2-carboxyethyl]carbamoylamino}-5-chloro-4-methylbenzenesulfonic acid, a pharmaceutically acceptable salt thereof, or a solvate thereof.

## BRIEF DESCRIPTION OF THE DRAWINGS

[Figure 1] Arrhenius plots (A: 0.1 mg/mL, pH 1.1, B: 0.1 mg/mL, pH 7.0, C: 10 mg/mL, pH 1.1, D: 10 mg/mL, pH 7.0).
[Figure 2-1] A graph showing a relationship between a serum iPTH concentration and given doses 48 hours after a single intravenous administration to bilaterally nephrectomized rats in Reference Example 2.
[Figure 2-2] A graph showing a relationship between a serum Ca concentration and administration doses 48 hours after a single intravenous administration to bilaterally nephrectomized rats in Reference Example 2.
[Figure 2-3] A graph showing results of comparing survival numbers of the bilaterally nephrectomized rats 48 hours after the single intravenous administration in Reference Example 2.
[Figure 2-4] A graph showing a histamine release rate from peritoneal mast cells upon administering respective compounds in Reference Example 4.
[Figure 2-5] A graph obtained by simultaneously fitting changes in plasma concentrations for the three given doses in P1 using a three-compartment model.
[Figure 2-6] A schematic view showing an indirect response model incorporating rebound.
[Figure 2-7] A graph of PK/PD analysis based on PI using the indirect response model incorporating rebound.
[Figure 2-8] A graph showing estimated PK and deduced EC50 value (> 1.4 ng/mL) in patients on dialysis.

## MODE FOR CARRYING OUT THE INVENTION

Hereinafter, the present invention will be described in detail.

The present invention provides a medicinal composition comprising 3-{[(2S)-2-amino-2-carboxyethyl]carbamoylamino}-5-chloro-4-methylbenzenesulfonic acid (hereinafter, may be referred to as "Compound A"), a pharmaceutically acceptable salt thereof, or a solvate thereof, in which the medicinal composition has a pH of 8.0 or less. Hereinafter, the medicinal composition may be referred to as a "medicinal composition of the present invention".

The medicinal composition according to some aspects of the present invention is used for treating secondary hyperparathyroidism with a patient developing or having the risk of developing secondary hyperparathyroidism, specifically, a patient with a chronic kidney disease on continuous dialysis. The medicinal composition is preferably used for long-term administration.

"Secondary hyperparathyroidism" refers to hyperparathyroidism that occurs from continuous presence of a factor stimulating the parathyroid glands, which is induced by abnormal bone mineral metabolism resulting from renal function disorders, and refers to a state where the serum PTH concentration prior to administration of the medicinal composition of the present invention is exceeding a certain range. A PTH concentration can be measured by various measurement methods, for example, as intact PTH (iPTH) which can be acquired by measuring only the full-length PTH, or as whole PTH which can be acquired by measuring only biologically active full-length PTH. Based on the reference values of dialysis patients which are defined for respective measurements, secondary hyperparathyroidism is diagnosed when the measured PTH values exceed a certain range. In general, secondary hyperparathyroidism is diagnosed in terms of iPTH value, specifically when iPTH exceeds 300 pg/ml, and in some cases when iPTH exceeds 240 pg/ml.

Here, in a case of hypoalbuminemia (albumin is 4 g/dl or less), a serum Ca concentration can be corrected by the following equation.

$$\text{Corrected serum Ca level [mg/dl]} = \text{Measured serum Ca level [mg/dl]} + 4 - \text{Serum albumin level [g/dl]}$$

"Treatment" of secondary hyperparathyroidism means to administer the medicinal composition of the present invention to a patient developing secondary hyperparathyroidism so as to lower the serum PTH concentration to be lower than the concentration prior to administration of the medicinal composition of the present invention, preferably to the reference value for dialysis patients. More preferably, the treatment means that the serum PTH concentration is not lowered beyond a lower limit value of the reference value for dialysis patients, and an upper limit value of the serum PTH concentration is lowered up to the reference value for dialysis patients. For example, the treatment means that the serum PTH concentration is regulated to a normal level. Moreover, at the same time as lowering the serum PTH concentration to the reference value for dialysis patients, it may also mean to suppress the progression of parathyroid hyperplasia and mineral metabolism disorders (especially, Ca and P) which are symptoms relating to secondary hyperparathyroidism, preferably to improve the symptoms from how they were before administration of the medicinal composition of the present invention, or to keep the parameters relative to the mineral metabolism disorder within the reference values for dialysis patients.

In the present specification, the term "treatment" is used in the meaning including "prevention". "Prevention" of secondary hyperparathyroidism means to administer the medicinal composition of the present invention to a patient whose serum PTH concentration is within the reference range of dialysis patients prior to administration of the medicinal composition of the present invention but who is at risk of developing secondary hyperparathyroidism due to renal function disorders so that the measured serum PTH concentration does not exceed the upper limit of the reference value for dialysis patients.

The term "side effects" refers to side effects that have been a problem of an existing drug having the same indications, specifically, digestive symptoms such as nausea and vomiting, hypersensitivity reaction, dysgeusia, hypocalcemia, exacerbation of heart failure caused by hypocalcemia, QT prolongation, numbness, a muscle spasm, sick feeling, arrhythmia, hypotension and a spasm.

The term "reduced side effects" means that occurrence of side effects caused by an existing drug having the same indications is lower than the existing drug when administration is conducted with a prescribed usage and dose. Specifically, it means that occurrence of the side effects is 20% or less, 15% or less, 10% or less, 5% or less, and preferably 1% or less in a patient given with the medicinal composition of the present invention.

The phrase "without manifesting significant accumulating property" means that even if the medicinal composition of the present invention is continuously administered (for 1 month or longer) with a defined usage and dose, the concentration of Compound A in the blood is not significantly increased in proportion to the period of administration.

The phrase "used for long-term administration" means that it is a medicinal composition with fewer cases of medication discontinuation, which is more adaptable to long-term administration as compared to an existing drug having the same indications (cinacalcet and etelcalcetide). Specifically, it refers to continuous administration of 1 year or longer.

The phrase "regulate to the normal level" means to regulate the serum PTH or Ca concentration to a level that

is judged to be clinically unproblematic by the physician, preferably, to be within the range of the reference values for dialysis patients defined for the respective inspection values. More preferably, it means that regulation to the above-mentioned level is conducted by administration within a defined usage and dose range without any break from medication during the period of administration.

The reference value of the serum iPTH concentration for dialysis patients is in a range of 60 pg/mL to 300 pg/mL, and preferably in a range of 150 pg/mL to 300 pg/mL or 60 pg/mL to 240 pg/mL.

The reference value of the serum Ca concentration for dialysis patients (corrected serum Ca concentration in case of hypoalbuminemia) is generally in a range of 8.4 mg/dl to 10.0 mg/dl.

Note that the normal level also includes cases where there is no need of discontinuing the administration even though the concentration may temporarily deviate from the above-described reference value range.

The medicinal composition according to the present invention comprises, as an active ingredient, Compound A, a pharmaceutically acceptable salt thereof, or a solvate thereof, and a pharmaceutically acceptable carrier such as a pharmaceutically acceptable nontoxic carrier. Compound A used in the present invention, the pharmaceutically acceptable salt thereof, or the solvate thereof is a compound represented by the following Formula (I), a pharmaceutically acceptable salt thereof, or a solvate thereof.

[Formula 1]

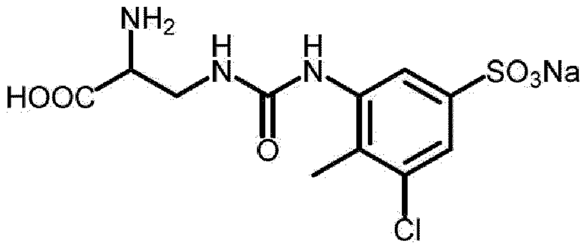

(I)

Compound A having the above structure, the pharmaceutically acceptable salt thereof, or the solvate thereof can be prepared by methods disclosed in WO 2011/108690 A and JP 2013-63971 A or methods equivalent thereto. A sodium salt of Compound A (Compound A1) can be prepared by a method in Reference Example 1 to be described below and a method equivalent thereto.

In a case where the medicinal composition according to the present invention is a liquid formulation, the medicinal composition according to the present invention comprises Compound A, a pharmaceutically acceptable salt thereof, or a solvate thereof, for example, at a concentration of 0.01 mg/mL to 10 mg/mL, preferably at a concentration of 0.01 mg/mL to 5 mg/mL, and more preferably at a concentration of 0.02 mg/mL to 2 mg/mL.

Compound A used for the present invention also comprises Compound A in a salt form. If Compound A, an active ingredient of the present invention, is in a salt form, the salt is a pharmaceutically acceptable salt or an edible salt. Examples of salts of an acidic group in Formula (I) include an ammonium salt, a salt with a metal such as sodium, potassium, calcium, magnesium, aluminum, or zinc, a salt with an organic amine such as triethylamine, ethanolamine, morpholine, pyrrolidine, piperidine, piperazine, or dicyclohexylamine, and a salt with a basic amino acid such as arginine or lysine. Examples of salts of a basic group in Formula (I) include a salt with an inorganic acid such as hydrochloric acid, sulfuric acid, phosphoric acid, nitric acid, or hydrobromic acid, a salt with an organic carboxylic acid such as acetic acid, trifluoroacetic acid, citric acid, benzoic acid, maleic acid, fumaric acid, tartaric acid, succinic acid, tannic acid, butyric acid, hibenzic acid, pamoic acid, enanthic acid, decanoic acid, teoclic acid, salicylic acid, lactic acid, oxalic acid, mandelic acid, or malic acid, and a salt with an organic sulfonic acid such as methanesulfonic acid, benzenesulfonic acid, or p-toluenesulfonic acid. These salts can be produced by making Compound A into contact with an acid or a base that can be used for producing a pharmaceutical product.

Preferably, it is a sodium salt of Compound A.

In the present invention, Compound A or a salt thereof may be an anhydride, and may form a solvate such as a hydrate or an alcohol adduct. As used herein, "solvation" refers to a phenomenon where solute molecules or ions strongly attract the surrounding solvent molecules and form one molecular cluster in a solution. For example, it is called hydration if the solvent is water. The solvate may be either a hydrate or a nonhydrate. A nonhydrate may use an alcohol (for example, methanol, ethanol, n-propanol), dimethylformamide, or the like.

Preferably, it is a hydrate of a sodium salt of Compound A.

If Compound A is obtained in a free form, it may be converted into a form of a salt, a hydrate thereof, or a solvate thereof that may result from Compound A, according to a conventional procedure.

Moreover, if Compound A is obtained as a salt, a hydrate, or a solvate thereof, it may be converted into a free

form of Compound A, according to a conventional procedure.

Compound A is intravenously administered as a medicinal composition comprising Compound A as an active ingredient. The method for applying such a medicinal composition is not particularly limited and the medicinal composition may be administered in a form of a commonly employed medicine formulation by mixing the active ingredient with a pharmaceutically acceptable nontoxic liquid carrier that is suitable for administration such as injection. The medicinal composition according to the present invention is usually a liquid formulation.

Examples of such a formulation include forms of liquid formulations such as a solution, a suspension, and an emulsion, and forms such as a lyophilized agent. These formulations can be prepared by a pharmaceutically common process.

Examples of the pharmaceutically acceptable nontoxic carrier include water (for example, water for injection), physiological saline, and monohydric or polyhydric alcohol (for example, glycerol or the like). In addition, if necessary, a common additive such as a pH adjuster, a stabilizer, an emulsifier, or a tonicity agent can also suitably be added.

The medicinal composition according to the present invention has a pH of 8.0 or less. A pH of the medicinal composition according to another embodiment of the present invention is, for example, 1.0 to 8.0, 1.5 to 7.5, 2.0 to 7.5, 3.0 to 7.5, 4.0 to 7.5, 5.0 to 7.5, 6.0 to 7.5, or 6.0 to 7.0. The medicinal composition according to a preferred aspect of the present invention has a pH of 5.0 to 7.5, and more preferably has a pH of 6.0 to 7.0.

In the present invention, excellent stability can be obtained by adjusting the pH of the medicinal composition as described above.

Here, it is found that the following decomposition product (1B) and decomposition product (1C) are produced from Compound A as main decomposition products.

[Formula 2]

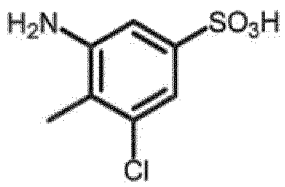

1B

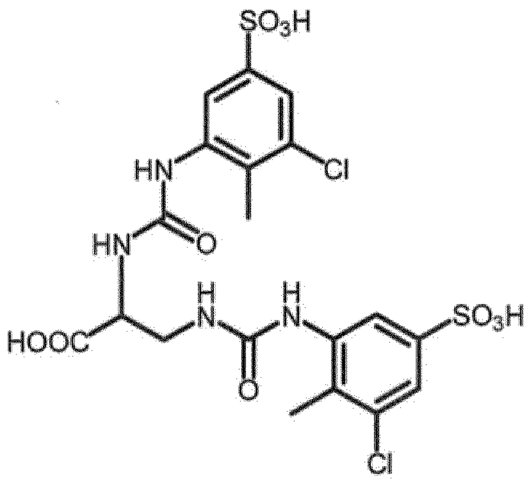

1C

Therefore, during storage, a production amount of each of the decomposition product (1B) and the decomposition product (1C) can be used as an index of stability. In the present invention, by adjusting the pH of the medicinal composition to 8.0 or less, the production of each of the decomposition product (1B) and the decomposition product (1C) can be suppressed during long-term storage. In the medicinal composition according to the present invention, the total production amount of the decomposition product (1B) and the decomposition product (1C) can be suppressed to preferably 6% by weight or less, and more preferably 5% by weight or less, with respect to the initial weight of Compound A in the medicinal composition when the medicinal composition is stored for a long period of time.

Here, the long-term storage means, for example, storage for 6 months or longer or 12 months or longer at 5°C; 6 months or longer at 15°C; 3 months or longer at 25°C; or 1 week or longer at 40°C, preferably storage for 6 months or longer or 12 months or longer at 5°C; 6 months or longer at 15°C; or 3 months or longer at 25°C, more preferably storage for 6 months or longer or 12 months or longer at 5°C or 6 months or longer at 15°C, and further preferably storage for 12 months or longer at 5°C or 6 months or longer at 15°C. The long-term storage is, for example, 30 months or shorter at 5°C; 12 months or shorter at 15°C; 6 months or shorter at 25°C; or 1 month or shorter at 40°C, in addition to being equal to or longer than each lower limit of the period.

In other words, the fact that the production of each of the decomposition product (1B) and the decomposition product (1C) can be suppressed during storage can mean that a residual ratio of Compound A, a pharmaceutically acceptable salt thereof, or a solvate thereof is high. The residual ratio of Compound A, the pharmaceutically acceptable

salt thereof, or the solvate thereof in the medicinal composition according to the present invention is preferably 94% by weight or more, and more preferably 95% by weight or more, with respect to the initial total weight of Compound A in the composition.

In the medicinal composition of the present invention, the pH can be adjusted by a pH adjuster and/or a buffer. In general, the "pH adjuster and/or the buffer" used in the present invention is a pH adjuster and/or a buffer used in a pharmaceutical formulation.

Examples of the "pH adjuster" used herein include, but are not limited to, an inorganic acid such as hydrochloric acid, sulfuric acid, and phosphoric acid, and a salt thereof, an organic acid such as acetic acid, citric acid, succinic acid, tartaric acid, lactic acid, and maleic acid, and a salt thereof, an inorganic base such as sodium hydroxide and ammonia water, and an organic base such as meglumine. The pH adjuster preferably used in the present invention is an organic acid such as acetic acid, citric acid, succinic acid, or tartaric acid, or a salt thereof, an inorganic acid such as hydrochloric acid or phosphoric acid, or a salt thereof, or inorganic bases such as sodium hydroxide and ammonia water. The pH adjuster particularly preferably used is citric acid, sodium citrate, acetic acid, sodium acetate, sodium monohydrogen phosphate, sodium dihydrogen phosphate, or disodium hydrogen phosphate. These pH adjusters may be used alone or in combination of two or more thereof. The pH adjuster may be one or a combination of two or more selected from phosphoric acid and salts thereof, and is preferably disodium hydrogen phosphate hydrate or sodium dihydrogen phosphate.

Examples of the "buffer" used herein include, but are not limited to, an inorganic acid such as phosphoric acid and salts thereof, an organic acid such as acetic acid, citric acid, tartaric acid, lactic acid, and maleic acid, and salts thereof, an amino acid such as histidine and arginine and salts thereof, and an organic base such as trometamol and meglumine. The buffer preferably used in the present invention is an organic acid such as acetic acid, citric acid, or tartaric acid, or salts thereof, or an amino acid such as histidine or arginine, or salts thereof. The buffer particularly preferably used is citric acid, sodium citrate, acetic acid, sodium acetate, sodium monohydrogen phosphate, sodium dihydrogen phosphate, or disodium hydrogen phosphate. These pH adjusters may be used alone or in combination of two or more thereof.

Either one or both of the pH adjuster and the buffer may be used.

In the medicinal composition of the present invention, a content of the pH adjuster and/or the buffer is appropriately determined depending on the type, desired pH value, and the like of the pH adjuster and/or the buffer, and the content of each of the pH adjuster and the buffer in the medicinal composition is, for example, 0.0001 to 30% by weight, preferably 0.001 to 20% by weight, and more preferably 0.005 to 10% by weight.

The medicinal composition of the present invention may comprise an additive that can be used in the pharmaceutical field, if necessary, in addition to the pH adjuster and/or the buffer.

The additive is not particularly limited, and examples thereof include a tonicity agent, a solubilizer, and a preservative.

Examples of the tonicity agent include, but are not limited to, sodium chloride, calcium chloride, potassium chloride, citric acid, sodium citrate, glycerin, concentrated glycerin, sodium hydrogen carbonate, sodium lactate, glucose, propylene glycol, macrogol, D-mannitol, phosphoric acid, sodium phosphate, potassium dihydrogen phosphate, sodium hydrogen phosphate, and sodium dihydrogen phosphate. The tonicity agent is preferably sodium chloride, D-mannitol, glycerin, concentrated glycerin, glucose, or propylene glycol. These pH adjusters may be used alone or in combination of two or more thereof.

Examples of the solubilizer include ethanol, ethylenediamine, capric acid, L-glutamic acid, L-lysine, calcium oxide, magnesium oxide, sorbitan sesquioleate, D-sorbitol, nicotinamide, propylene glycol, polysorbate 80, and lauromacrogol.

Examples of the preservative include phenol, sodium edetate, benzalkonium chloride, chlorocresol, chlorobutanol, sodium salicylate, ethyl parahydroxybenzoate, and butyl parahydroxybenzoate.

The medicinal composition of the present invention may comprise one or a combination of two or more of the additives.

In general, the medicinal composition of the present invention is filled and stored in a container. Examples of the container include a glass container and a plastic container. Examples of a material of the plastic container include olefin-based resins such as polyethylene, polypropylene, and cyclic polyolefin. The shape of the container is not particularly limited, but examples thereof include a vial, a syringe, a bag, and a bottle. Therefore, the present invention includes a formulation in which a medicinal composition comprising Compound A and having a pH of 8.0 or less is filled in a vial, a syringe, a bag, or a bottle.

In general, the medicinal composition of the present invention is stored at 2 to 8°C (for example, 5°C).

In the medicinal composition of the present invention, Compound A is intravenously administered at the end of dialysis with a daily dose for adults selected from doses of 0.01 mg, 0.025 mg, and 0.05 mg or more, and selected from doses of 2.5 mg, 0.8 mg, 0.4 mg, 0.3 mg, and 0.2 mg or less, preferably 0.025 mg to 0.8 mg, more preferably 0.025 to 0.4 mg, and further preferably 0.05 to 0.2 mg.

"A daily dose" of Compound A "for adults selected from doses of 0.01 mg, 0.025 mg, and 0.05 mg or more, and selected from doses of 2.5 mg, 0.8 mg, 0.4 mg, 0.3 mg, and 0.2 mg or less" refers to any dose in a range of 0.01 mg to 2.5 mg, 0.01 mg to 0.8 mg, 0.01 mg to 0.4 mg, 0.01 mg to 0.3 mg, 0.01 mg to 0.2 mg, 0.025 mg to 2.5 mg, 0.025 mg to 0.8 mg, 0.025 mg to 0.4 mg, 0.025 mg to 0.3 mg, 0.025 mg to 0.2 mg, 0.05 mg to 2.5 mg, 0.05 mg to 0.8 mg, 0.05 mg to 0.4 mg, 0.05 mg to 0.3 mg, or 0.05 mg to 0.2 mg. If Compound A is a solvate, the dose refers to an amount of Compound A in terms of a nonsolvate. If Compound A is a solvate of a salt, the dose refers to an amount of free Compound A in terms of a nonsolvate. In a case where the patient is Japanese, the dose is preferably 0.025 mg to 0.8 mg. The preferred dose may vary depending on races. For example, Caucasoid and Australoid, generally require a higher dose than that the preferred dose for the Mongoloid such as Japanese.

Since the medicinal composition of the present invention is administered at the time of dialysis (upon dialysis), if the general dialysis schedule consists of three sessions a week, the medicinal composition of the present invention is administered upon each dialysis session. In this case, assuming that the beginning of a week is Day 1, dialysis is conducted, for example, on Days 1, 3, and 5, upon which the medicinal composition of the present invention is administered, and the same schedule is repeated from next week and on. If the dialysis schedule consists of 4 sessions a week, or if the dialysis is more than 4 sessions are temporarily conducted due to the patient condition or the like, the medicinal composition of the present invention is administered following the same schedule of 4 or more dialysis sessions a week. Preferably, the medicinal composition of the present invention is administered at the end of each dialysis session in the dialysis schedule of 3 to 5 sessions a week.

Here, the phrase "the end of dialysis" means immediately before the end of dialysis, specifically, when the blood is returned immediately before the end of dialysis. The phrase "intravenously administered" means to directly administer the drug into the vein, but, for dialysis patients, it is preferable to administer the drug from the venous side of the dialysis circuit.

More preferably, the drug is infused into the venous side of the dialysis circuit upon returning the blood at the end of dialysis.

The medicinal composition of the present invention may include an insert instructing the use thereof inside the package. An example of such an insert includes so-called instructions explaining the use, efficacy, administration method and the like.

In accordance with symptoms of the patient, the medicinal composition of the present invention may be used in combination with a calcium agent or a vitamin D formulation. The usage and dose of the calcium agent or the vitamin D formulation used in combination can appropriately be determined according to the blood Ca concentration.

## EXAMPLES

Hereinafter, the present invention will be described specifically by means of examples, although the present invention should not be interpreted to be limited to these examples.

Examples of Compound A1 and a synthesis method thereof were described in Reference Example 1 to be described below.

Here, in Examples 1A, 1B, 4A, and 4B, a pH of each of formulations was measured after preparation of the formulation using a pH meter (manufactured by Mettler Toledo, trade name: HM-30G).

### Example 1A

Compound A1 was dissolved in each of the buffer solutions so that a concentration was 0.1 mg/mL. Each of these solutions was dispensed into a glass vial and stored at 5°C. The preparation method of each of the buffer solutions and the storage period of the Compound A1 solution are shown in Table 1 below. After the storage, a residual ratio was calculated according to a test method described below.

[Table 1]

Table 1 Preparation method of each of buffer solutions and storage period of Compound A1 solution

| pH | Preparation method | Storage period (hours) |
|---|---|---|
| 1.1 | Mixing 100 mL of 0.2 M aqueous hydrochloric acid solution with 100 mL of water | 24, 113, 161 |
| 2.0 | Mixing 26.0 mL of 0.2 M aqueous hydrochloric acid solution with 174.0 mL of 0.2 M aqueous potassium chloride solution | |
| 3.0 | Mixing 162.2 mL of 0.1 M aqueous citric acid solution with 37.8 mL of 0.2 M aqueous disodium hydrogen phosphate solution | |
| 4.0 | Mixing 123.8 mL of 0.1 M aqueous citric acid solution with 76.2 mL of 0.2 M aqueous disodium hydrogen phosphate solution | |
| 5.0 | Mixing 98.0 mL of 0.1 M aqueous citric acid solution with 102.0 mL of 0.2 M aqueous disodium hydrogen phosphate solution | |
| 6.0 | Mixing 75.0 mL of 0.1 M aqueous citric acid solution with 125.0 mL of 0.2 M aqueous disodium hydrogen phosphate solution | |
| 7.0 | Mixing 35.6 mL of 0.1 M aqueous citric acid solution with 164.4 mL of 0.2 M aqueous disodium hydrogen phosphate solution | |

Test method: In the solution after the storage, the residual ratio was analyzed according to a normal method using a liquid chromatography apparatus Prominence (manufactured by Shimadzu Corporation) and an ultraviolet absorptiometer (measurement wavelength: 254 nm, Prominence (manufactured by Shimadzu Corporation)). At this time, SUMIPAX ODS (3 mm × 150 mm, 5 μm, manufactured by SCAS) was used as a column.

The residual ratio (%) of Compound A1 during each storage period at 5°C was calculated as follows.

$$\text{Residual ratio (\%) of Compound A1 during each storage period} = \text{peak area of Compound A1 in sample solution} / \text{sum of peak area of sample solution} \times 100$$

The results are shown in Table 2 below.

[Table 2]

Table 2 Residual ratio at 5°C

| pH | Residual ratio (%) | | |
|---|---|---|---|
| | 24 hours | 113 hours | 161 hours |
| 1.1 | 100 | 100 | 100 |
| 2.0 | 100 | 100 | 100 |
| 3.0 | 100 | 100 | 100 |
| 4.0 | 100 | 100 | 100 |
| 5.0 | 100 | 100 | 100 |
| 6.0 | 100 | 100 | 100 |
| 7.0 | 100 | 100 | 100 |

According to the present example, the formulation comprising Compound A1 and having a pH of 1.1 to 7.0 was stable at 5°C.

Example 1B

According to Table 3 below, Compound A1 was dissolved in each of the buffer solutions at 2 concentrations. Each of these solutions was dispensed into a glass vial and stored at 5°C. The preparation method of each of the buffer solutions and the storage period of the Compound A1 solution are shown in Table 3 below. After the storage, a residual ratio of Compound A1 was calculated according to a test method described below.

[Table 3]

Table 3 Preparation method of each of buffer solutions and storage period of Compound A1 solution

| Concentration | pH | Preparation method | Storage period (hours) |
|---|---|---|---|
| 0.1 mg/mL | 1.1 | Mixing 100 mL of 0.2 M aqueous hydrochloric acid solution with 100 mL of water | 23, 141, 570 |
| 10. mg/mL | | | |
| 0.1 mg/mL | 7.0 | Mixing 35.6 mL of 0.1 M aqueous citric acid solution with 164.4 mL of 0.2 M aqueous disodium hydrogen phosphate solution | |
| 10. mg/mL | | | |

Test method: In the solution after the storage, the residual ratio was analyzed according to a normal method using a liquid chromatography apparatus Prominence (manufactured by Shimadzu Corporation) and an ultraviolet absorptiometer (measurement wavelength: 254 nm, Prominence (manufactured by Shimadzu Corporation)). At this time, SUMIPAX ODS (3 mm × 150 mm, 5 μm, manufactured by SCAS) was used as a column.

The residual ratio (%) of Compound A1 during each storage period at 5°C was calculated as follows.

$$\text{Residual ratio (\%) of Compound A1 during each storage period} = \text{peak area of Compound A1 in sample solution} / \text{sum of peak area of sample solution} \times 100$$

The results are shown in Table 4 below.

[Table 4]

Table 4 Residual ratio at 5°C

| Concentration (mg/mL) | pH | Residual ratio (%) | | |
|---|---|---|---|---|
| | | 23 hours | 141 hours | 570 hours |
| 0.1 | 1.1 | 100 | 100 | 100 |
| 10 | | 100 | 100 | 100 |
| 0.1 | 7.0 | 100 | 100 | 100 |
| 10 | | 100 | 100 | 100 |

According to the present example, the formulation comprising Compound A1 and having a pH of 1.1 and pH of 7.0 was stable at 5°C regardless of the concentration of Compound A1 in the formulation.

It was found from the results of Examples 1A and 1B that the formulation comprising Compound A1 was stable when the pH was 8.0 or less regardless of the concentration of Compound A1 in the formulation.

Example 2

Compound A1 weighed so as to have the composition shown in Table 5 below and each of the buffer solutions

were dissolved in water for injection to obtain a uniform solution. These solutions were filled in glass ampoules and sealed. The pH in each of Examples 2-1 to 2-4 and Comparative Example was measured after dissolution using a pH meter S20 (manufactured by Mettler Toledo).

[Table 5]

Table 5

| Components | Example 2-1 pH 3.0 | Example 2-2 pH 4.5 | Example 2-3 pH 6.0 | Example 2-4 pH 7.0 | Comparative Example pH 8.5 |
|---|---|---|---|---|---|
| CompoundAl | 1.0 mg | 1.0 mg | 1.0 mg | 1.0 mg | 1.0 mg |
| 10 mM citric acid buffer solution | Appropriate amount | - | - | - | - |
| 10 mM acetic acid buffer solution | - | Appropriate amount | - | - | - |
| 10 mM phosphoric acid buffer solution | - | - | Appropriate amount | Appropriate amount | Appropriate amount |
| Water for injection | Appropriate amount | Appropriate amount | Appropriate amount | Appropriate amount | Appropriate amount |
| Total | 1 mL | 1 mL | 1 mL | 1 mL | 1 mL |

Each of the solutions filled in the glass ampoule was stored at 40°C for 1 month, and then the residual ratio of Compound A1 and the production of each of decomposition products were evaluated. The evaluation was performed as follows using liquid chromatography.

Test method: In the solution after the storage, the residual ratio was analyzed according to a normal method using a liquid chromatography apparatus (ACQUITY UPLC H-Class; measurement wavelength: 210 nm). At this time, XSELECT CSH C18 (2.1 mm × 100 mm, 3.5 μm, manufactured by Waters Corporation) was used as a column.

Calculation for obtaining the residual ratio (%) of Compound A1 and the residual ratio (%) of each of the decomposition products during each storage period is as follows.

$$\text{Residual ratio (\%) of Compound A1} = \text{peak area of Compound A1 in sample solution/ sum of peak area of sample solution} \times 100$$

$$\text{Each of decomposition products (\%)} = \text{peak area of each of decomposition products in sample solution/ sum of peak area of sample solution} \times 100$$

The results are shown in Table 6 below. As shown in Table 6, at the pH of 3.0 to 7.0, the production of each of the decomposition products 1B and 1C was suppressed to be below the threshold (that is, the total of the decomposition products 1B and 1C was 6% or less) and thus the formulation was stable, whereas at the pH of 8.5, the production of each of the decomposition products 1B and 1C was exceeded the threshold and thus the formulation was unstable. From the above results, when the pH was 3.0 to 7.0, excellent storage stability was obtained even at 40°C.

[Table 6]

Table 6

| Storage period | Evaluation item | Example 2-1<br>pH 3.0 | Example 2-2<br>pH 4.5 | Example 2-3<br>pH 6.0 | Example 2-4<br>pH 7.0 | Comparative Example<br>pH 8.5 |
|---|---|---|---|---|---|---|
| At time of beginning | Residual ratio (%) | 99.8 | 99.8 | 99.8 | 99.7 | 99.83 |
| | 1B (%) | - | - | 0.06 | - | - |
| | 1C (%) | - | - | - | - | - |
| 1 month | Residual ratio (%) | 96.4 | 96.5 | 96.4 | 95.7 | 93.43 |
| | 1B (%) | 3.03 | 3.38 | 2.98 | 1.98 | 0.98 |
| | 1C (%) | - | - | 0.36 | 1.90 | 5.24 |

According to the present example, it was confirmed that the formulation comprising Compound A1 and having a pH of 8.0 or less was stable at 40°C for 1 month. Since the stability of the formulation is greatly affected by the temperature during storage, a storage stability test was performed under various temperature conditions.

Example 3

Compound A1 weighed so as to have the composition shown in Table 7 below and each of the buffer solutions were dissolved in water for injection to obtain a uniform solution. These solutions were filled in glass ampoules and sealed. Each pH in Example 3 was measured during each storage period using a pH meter HM-30R (manufactured by DKK-TOA CORPORATION).

[Table 7]

Table 7

| Components | 0.1 mg/mL solution | 10 mg/mL solution |
|---|---|---|
| Compound A1 | 0.1 mg | 10 mg |
| Sodium chloride | 9 mg | 7.4 mg |
| 10 mM phosphoric acid buffer solution | Appropriate amount | Appropriate amount |
| Total | 1 mL | 1 mL |

The residual ratio of Compound A1 and the production of each of the decomposition products at 5°C, 15°C, and 25°C were evaluated according to a test method described below.

Test method: In the solution after the storage, the residual ratio was analyzed according to a normal method using a liquid chromatography apparatus (ACQUITY UPLC H-Class; measurement wavelength: 210 nm). At this time, XSELECT CSH C18 (2.1 mm × 100 mm, 3.5 μm, manufactured by Waters Corporation) was used as a column.

Calculation for obtaining the residual ratio (%) of Compound A1 and each of the decomposition products (%) during each storage period is as follows.

$$\text{Residual ratio (\%) of Compound A1} = \text{peak area of Compound A1 in sample solution} / \text{sum of peak area of sample solution} \times 100$$

$$\text{Each of decomposition products (\%)} = \text{peak area of each of decomposition products in sample solution/ sum of peak area of sample solution} \times 100$$

The results of the formulation comprising Compound A1 at a concentration of 10 mg/mL are shown in Table 8, and the results of the formulation comprising Compound A1 at a concentration of 0.1 mg/mL are shown in Table 9. As shown in Table 8 and Table 9, in the case where each of the formulations comprising Compound A1 in the pH range of 6.4 to 6.9 was stored under the condition of 5 to 25°C (further 60%RH at 25°C), excellent storage stability was obtained for 30 months when being stored at 5°C, was obtained for 12 months when being stored at 15°C, and was obtained for 6 months when being stored at 25°C, regardless of the concentration of Compound A1.

It was found from the present example that the formulation having the pH of 8.0 or less was stable at the condition of 5 to 25°C regardless of the concentration of Compound A1.

[Table 8]

Table 8

| Storage condition | Storage period | Compound A1 formulation (10 mg/mL) | | | | |
|---|---|---|---|---|---|---|
| | | 0M | 3M | 6M | 12M | 30M |
| 5°C | pH | 6.4 | 6.4 | 6.5 | 6.5 | 6.5 |
| | Residual | 99.91 | 99.89 | 99.86 | 99.84 | 99.73 |
| | ratio (%) | | | | | |
| | 1B (%) | <0.03 | <0.03 | <0.03 | <0.03 | 0.04 |
| | 1C (%) | <0.03 | <0.03 | 0.04 | 0.07 | 0.15 |
| 15°C | pH | 6.4 | - | 6.5 | 6.5 | - |
| | Residual ratio (%) | 99.91 | - | 99.62 | 99.38 | - |
| | 1B (%) | <0.03 | - | 0.07 | 0.13 | - |
| | 1C (%) | <0.03 | - | 0.20 | 0.37 | - |
| 25°C/60%RH | pH | 6.4 | 6.4 | 6.8 | - | - |
| | Residual ratio (%) | 99.91 | 99.04 | 98.16 | - | - |
| | 1B (%) | <0.03 | 0.24 | 0.48 | - | - |
| | 1C (%) | <0.03 | 0.55 | 0.96 | - | - |

[Table 9]

Table 9

| Storage condition | Storage period | Compound A1 formulation (0.1 mg/mL) | | | | |
|---|---|---|---|---|---|---|
| | | 0M | 3M | 6M | 12M | 30M |
| 5°C | pH | 6.5 | 6.5 | 6.5 | 6.7 | 6.6 |
| | Residual ratio (%) | 99.91 | 99.90 | 99.88 | 99.87 | 99.77 |
| | 1B (%) | <0.03 | 0.03 | 0.04 | 0.07 | 0.14 |
| | 1C (%) | <0.03 | <0.03 | <0.03 | <0.03 | 0.03 |

(continued)

| Storage condition | Storage period | Compound A1 formulation (0.1 mg/mL) | | | | |
|---|---|---|---|---|---|---|
| | | 0M | 3M | 6M | 12M | 30M |
| 15°C | pH | 6.5 | - | 6.9 | 6.9 | - |
| | Residual ratio (%) | 99.91 | - | 99.67 | 99.49 | - |
| | 1B (%) | <0.03 | - | 0.22 | 0.39 | - |
| | 1C (%) | <0.03 | - | <0.03 | 0.07 | - |
| 25°C/60%RH | pH | 6.5 | 6.7 | 6.6 | - | - |
| | Residual ratio (%) | 99.91 | 99.20 | 98.38 | - | - |
| | 1B (%) | <0.03 | 0.67 | 1.34 | - | - |
| | 1C (%) | <0.03 | 0.07 | 0.17 | - | - |

In order to further estimate the stability of the formulation comprising Compound A1, the following test was performed.

Example 4A

Compound A1 was dissolved in each of the buffer solutions shown in Table 10 so that a concentration was 0.1 mg/mL. Each of these solutions was dispensed into a glass vial and stored at 60°C. The preparation method of each of the buffer solutions and the storage period of the Compound A1 solution are shown in Table 10 below. After the storage, a residual ratio was calculated according to a test method described below.

[Table 10]

Table 10 Preparation method of each of buffer solutions and storage period of Compound A1 solution

| pH | Preparation method | Storage period (hours) |
|---|---|---|
| 1.1 | Mixing 100 mL of 0.2 M aqueous hydrochloric acid solution with 100 mL of water | 24, 113, 161 |
| 2.0 | Mixing 26.0 mL of 0.2 M aqueous hydrochloric acid solution with 174.0 mL of 0.2 M aqueous potassium chloride solution | |
| 3.0 | Mixing 162.2 mL of 0.1 M aqueous citric acid solution with 37.8 mL of 0.2 M aqueous disodium hydrogen phosphate solution | |
| 4.0 | Mixing 123.8 mL of 0.1 M aqueous citric acid solution with 76.2 mL of 0.2 M aqueous disodium hydrogen phosphate solution | |
| 5.0 | Mixing 98.0 mL of 0.1 M aqueous citric acid solution with 102.0 mL of 0.2 M aqueous disodium hydrogen phosphate solution | |
| 6.0 | Mixing 75.0 mL of 0.1 M aqueous citric acid solution with 125.0 mL of 0.2 M aqueous disodium hydrogen phosphate solution | |
| 7.0 | Mixing 35.6 mL of 0.1 M aqueous citric acid solution with 164.4 mL of 0.2 M aqueous disodium hydrogen phosphate solution | |

Test method: In the solution after the storage, the residual ratio was analyzed according to a normal method using a liquid chromatography apparatus Prominence (manufactured by Shimadzu Corporation) and an ultraviolet absorptiometer (measurement wavelength: 254 nm, Prominence (manufactured by Shimadzu Corporation)). At this time, SUMIPAX ODS (3 mm × 150 mm, 5 μm, manufactured by SCAS) was used as a column.
The calculation for obtaining the residual ratio (%) of Compound A1 during each storage period is as follows.

$$\text{Residual ratio (\%) of Compound A1 during each storage period} = \text{peak area of Compound A1 in sample solution/peak area of Compound A1 in standard solution} \times 100$$

Here, "Compound A1 in standard solution" refers to a solution obtained by dissolving Compound A1 in each of the buffer solutions shown in Table 10 so that a concentration was 0.1 mg/mL, and then storing the solution at 5°C. It was clear from the results of Example 1 that the solution of Compound A1 was stable when being stored at 5°C, and thus, the solution was used as the standard solution.

[Table 11]

Table 11 Residual ratio after storage at 60°C

| pH | Residual ratio (%) | | |
|---|---|---|---|
| | 24 hours | 113 hours | 161 hours |
| 1.1 | 97.8 | 92.7 | 90.2 |
| 2.0 | 98.1 | 92.4 | 90.1 |
| 3.0 | 98.3 | 91.5 | 88.9 |
| 4.0 | 98.1 | 91.5 | 88.5 |
| 5.0 | 98.0 | 91.3 | 88.0 |
| 6.0 | 98.1 | 91.5 | 88.3 |
| 7.0 | 98.2 | 91.0 | 87.8 |

The reaction rate constant at 60°C was calculated using the obtained residual ratio. The results are shown in Table 12. The reaction rate constant at 60°C was not significantly changed even when the pH of the formulation was changed.

[Table 12]

Table 12 Reaction rate constant at 60°C

| pH | Reaction rate constant at 60°C |
|---|---|
| 1.1 | $6.3 \times 10^{-4}$ |
| 2.0 | $6.5 \times 10^{-4}$ |
| 3.0 | $7.5 \times 10^{-4}$ |
| 4.0 | $7.6 \times 10^{-4}$ |
| 5.0 | $7.9 \times 10^{-4}$ |
| 6.0 | $7.8 \times 10^{-4}$ |
| 7.0 | $8.2 \times 10^{-4}$ |

Example 4B

According to Table 13, Compound A1 was dissolved at 2 concentrations in each of the buffer solutions having a pH at each of both ends of Example 4A. Each of these solutions was dispensed into a glass vial and stored at each of 50°C, 60°C, and 70°C. The preparation method of each of the buffer solutions and the storage period of the Compound A1 solution are shown in Table 13 below. After the storage, a residual ratio was calculated according to a test method

described below.

[Table 13]

Table 13 Preparation method of each of buffer solutions and storage period of Compound A1 solution

| Concentration | pH | Preparation method | Storage period (hours) |
|---|---|---|---|
| 0.1 mg/mL | 1.1 | Mixing 100 mL of 0.2 M aqueous hydrochloric acid solution with 100 mL of water | 23, 141, 570 |
| 10. mg/mL | | | |
| 0.1 mg/mL | 7.0 | Mixing 35.6 mL of 0.1 M aqueous citric acid solution with 164.4 mL of 0.2 M aqueous disodium hydrogen phosphate solution | |
| 10. mg/mL | | | |

Test method: In the solution after the storage, the residual ratio was analyzed according to a normal method using a liquid chromatography apparatus Prominence (manufactured by Shimadzu Corporation) and an ultraviolet absorptiometer (measurement wavelength: 254 nm, Prominence (manufactured by Shimadzu Corporation)). At this time, SUMIPAX ODS (3 mm × 150 mm, 5 μm, manufactured by SCAS) was used as a column.

The calculation for obtaining the residual ratio (%) of Compound A1 during each storage period is as follows.

$$\text{Residual ratio (\%) of Compound A1 during each storage period} = \text{peak area of Compound A1 in sample solution/peak area of Compound A1 in standard solution} \times 100$$

Here, "Compound A1 in standard solution" refers to a solution obtained by dissolving Compound A1 in each of the buffer solutions shown in Table 13 so that a concentration was 0.1 mg/mL, and then storing the solution at 5°C. It was clear from the results of Example 1 that the solution of Compound A1 was stable when being stored at 5°C, and thus, the solution was used as the standard solution.

The results thereof are shown in Table 14.

[Table 14]

Table 14 Residual ratio after storage

| pH | Concentration (mg/mL) | Storage temperature (°C) | Residual ratio (%) | | |
|---|---|---|---|---|---|
| | | | 23 hours | 141 hours | 570 hours |
| 1.1 | 0.1 | 50 | 98.9 | 96.9 | 92.3 |
| | | 60 | 97.8 | 92.0 | 76.7 |
| | | 70 | 94.5 | 76.8 | 35.5 |
| | 10 | 50 | 96.2 | 96.4 | 89.5 |
| | | 60 | 96.4 | 95.6 | 73.4 |
| | | 70 | 92.5 | 84.2 | 36.5 |

(continued)

| pH | Concentration (mg/mL) | Storage temperature (°C) | Residual ratio (%) | | |
|---|---|---|---|---|---|
| | | | 23 hours | 141 hours | 570 hours |
| 7.0 | 0.1 | 50 | 99.6 | 97.7 | 90.9 |
| | | 60 | 98.2 | 89.2 | 63.9 |
| | | 70 | 93.8 | 68.0 | 21.1 |
| | 10 | 50 | 100.0 | 98.0 | 89.0 |
| | | 60 | 97.2 | 86.3 | 61.0 |
| | | 70 | 91.7 | 64.4 | 22.2 |

The reaction rate constant at each of the storing temperatures was calculated using the obtained residual ratio. The Arrhenius plot was performed to calculate the reaction rate constant ($h^{-1}$) at an arbitrary temperature. As an example, the residual ratio estimation at 25°C is shown in Table 15 and described in Fig. 1.

[Table 15]

Table 15 Residual ratio estimation at 25°C

| pH | Concentration (mg/mL) | Kobs ($hr^{-1}$) | Residual ratio (%) | | |
|---|---|---|---|---|---|
| | | | 3M | 6M | 12M |
| 1.1 | 0.1 | $3.01 \times 10^{-6}$ | 99.4 | 98.7 | 97.4 |
| | 10 | $5.55 \times 10^{-6}$ | 98.8 | 97.6 | 95.3 |
| 7.0 | 0.1 | $3.10 \times 10^{-6}$ | 99.3 | 98.7 | 97.4 |
| | 10 | $5.73 \times 10^{-6}$ | 98.8 | 97.6 | 95.2 |

As shown in Table 15, it was found that the formulation comprising Compound A1 and having a pH of 1.1 and 7.0 had excellent storage stability over 12 months even under the condition of 25°C.

Reference Example 1

Synthesis of sodium 3-{[(2S)-2-amino-2-carboxyethyl]carbamoylamino}-5-chloro-4-methylbenzenesulfonate (Compound A1)

(Step 1)

Synthesis of 3-({[(2S)-2-amino-3-methoxy-3-oxopropyl]carbamoyl}amino)-5-chloro-4-methylbenzene-1-sulfonic acid

To 5 g (22.56 mmol) of 3-amino-5-chloro-4-methylbenzenesulfonic acid (ACTS), 37.5 mL (7.5 L/kg vs ACTS) of acetonitrile, 3.81 mL (47.38 mmol, 2.1 eq.) of pyridine were added and stirred at 25°C. 2.99 mL (23.68 mmol, 1.05 eq.) of $ClCO_2Ph$ was dropped into the resultant, stirring was performed for 30 minutes, and thereafter the end of carbamate reaction was confirmed by HPLC. 5.92 g (23.23 mmol, 1.03 eq.) of 3-amino-N-(tert-butoxycarbonyl)-L-alanine methyl ester hydrochloride was added, 9.75 mL (69.93 mmol, 3.1 eq.) of triethylamine was dropped into the resultant, and stirring was performed at 25°C for 3 hours. 0.4 g (1.58 mmol, 0.07 eq.) of 3-amino-N-(tert-butoxycarbonyl)-L-alanine methyl ester hydrochloride and 0.22 mL (1.58 mmol, 0.07 eq.) of triethylamine were further added, and the end of urea-forming reaction was confirmed by HPLC. 7.32 mL (112.8 mmol, 5.0 eq.) of methanesulfonic acid was added, and the resultant was heated to 50°C and stirred for 4 hours. After confirming the end of deprotection by HPLC, the resultant was cooled to 25°C and added with 37.5 mL (7.5 L/kg) of acetonitrile and 7.5 mL (1.5 L/kg) of water to allow deposition of a solid. The resultant was cooled to 5°C and matured for 16 hours. The deposited solid was filtrated under reduced pressure, washed with 20 mL (4.0 L/kg) of water/acetonitrile (1/2), and then dried under reduced pressure at 40°C for

5 hours to obtain 7.72 g of a target compound as a white solid (net 7.20 g, 87.3%).

$^1$H-NMR (400MHz, DMSO-d6): δ 8.39 (bs, 3H), 8.16 (d, 1H, J=1.2Hz), 7.90 (d, 1H, J=1.6Hz), 7.28 (d, 1H, J=1.6Hz), 6.78 (t, 1H, J=5.6Hz), 4.20-4.10 (m, 1H), 3.77(s, 3H), 3.70-3.60 (m, 1H), 3.55-3.45 (m, 1H), 2.21 (s, 3H)

HRMS (FAB $^-$):calcd for m/z 364.0369(M-H), found m/z 364.0395(M-H)

(Step 2)

(2) Synthesis of sodium 3-{[(2S)-2-amino-2-carboxyethyl]carbamoylamino}-5-chloro-4-methylbenzenesulfonate

To 10.64 g (net 10.0g, 27.34 mmol) of the compound obtained in Step 1, 18 mL (1.8 L/kg vs compound of Step 1) of water was added and the resultant was stirred at 8°C. 3.42 mL (57.41 mmol, 2.1 eq.) of a 48% aqueous sodium hydroxide solution was dropped into the resultant, washing was performed with 1.0 mL (1.0 L/kg) of water, and then stirring was performed at 8°C for 15 minutes. After confirming the end of hydrolysis by HPLC, the resultant was heated to 25°C, and about 3.55 mL of 48% HBr aq. was added thereto to control the pH to 5.8. 65 mL (6.5 L/kg) of isopropyl alcohol was dropped into the resultant. After confirming deposition of the target compound, the resultant was matured for 1 hour. 81 mL (8.1 L/kg) of isopropyl alcohol was dropped into the resultant and matured at 8°C overnight. The deposited solid was filtrated under reduced pressure, washed with 20 mL (2.0 L/kg) of isopropyl alcohol, and dried under reduced pressure at 40°C for 4 hours to obtain 10.7 g of a target compound as a white solid (net 9.46 g, 92.6%).

$^1$H-NMR (400MHz, DMSO-d6): δ8.76 (s, 1H), 7.91 (d, 1H, J=1.6Hz), 8.00-7.50 (bs, 2H), 7.24 (d, 1H, J=1.6Hz), 7.20(t, 1H, J=5.6Hz), 3.58-3.54(m, 1H), 3.47-3.43(m, 1H), 3.42-3.37(m, 1H), 2.23(s, 3H)

Reference Example 2: Effect of Compound A1 on serum Ca concentration and serum iPTH concentration in bilaterally nephrectomized rats

After quarantine and acclimatization periods of 6 days, fifty-one rats (Crl: CD (SD), male) aged 7 weeks were fed a 100% sucrose diet ad libitum from four days before the surgery. On the day of the surgery, skin incisions were made on the back at the sites of the right and left kidneys under isoflurane anesthesia and the renal capsules were removed to ligate the renal arteries and veins and the ureters using threads before removing the right and left kidneys. A penicillin solution was given upon suturing, and 5 mL of physiological saline was intraperitoneally administered before returning the rats into the breeding cages.

On the day of administering the test substance, for example, a day after the bilaterally nephrectomy, approximately 100 μL of blood was collected from the tail vein without anesthesia using a capillary tube, which was centrifuged in a high speed refrigerated microcentrifuge (10,000 rpm, 5min, 4°C) to separate the serum. Thereafter, the serum Ca concentration was measured with a dri-chemanalyzer (model number: FUJI DRI-CHEM 7000, manufacturer: FUJI FILM Medical Co., Ltd.) to select individuals with a serum Ca concentration of 8.0 mg/dL or more and less than 14.0 mg/dL.

On a day after the bilaterally nephrectomy, the given doses were calculated based on the weight of the selected animals on that day. Group 1 was given a medium (physiological saline), Groups 2, 3 and 4 were given the Compound A1 solution (0.3, 3 and 30 mg/mL, respectively), and Groups 5, 6, 7 and 8 (5 rats per group) were given the comparative compound (0.3, 1, 3 and 10 mg/mL, respectively), each given a single dose of 1 ml/kg from the tail vein. Prior to the administration (0 hours), and 24 and 48 hours after the administration, approximately 300 μL of blood was collected without anesthesia from the tail vein using capillary tubes for obtaining the serum. The blood for obtaining the serum was left to stand at room temperature, and centrifuged in a high speed refrigerated microcentrifuge (10,000 rpm, 5 min, 4°C) within a period of 30 minutes to 2 hours after the blood collection to collect the serum.

Thereafter, the serum Ca was analyzed with COBAS analyzer (Model number: COBAS INTEGRA 400 plus, manufacturer: Roche Diagnostics K.K.), and the remaining serum was stored in an ultra-low freezer (temperature set to -80 ± 15°C) until the day of iPTH measurement. On the day of iPTH measurement, the serum was melted at room temperature for measurement.

(Compound A1 solutions)

A 30 mg/ml Compound A1 solution (338 mg of Compound A1 dissolved in 10 ml of physiological saline) was diluted with physiological saline to prepare 0.3 mg/ml and 1 mg/ml Compound A1 solutions.

(Here, the present invention can also include an aspect excluding the Compound A1 solution specifically described in each of Reference Examples 2 to 4.)

(Comparative compound solutions)

149 mg of etelcalcetide (Ac-c(C) arrrar-$NH_2$) TFA salt (WO 2011/014707 A) was prepared to have pH of 6 to

8 with 7 ml of physiological saline and a 0.5N aqueous NaOH solution, and physiological saline was further added thereto to make 10 ml. The prepared 10 mg/ml comparative compound solution was diluted with physiological saline to obtain 0.3 mg/ml and 1 mg/ml comparative compound solutions.

(Results)

(1) Serum iPTH concentration in bilaterally nephrectomized rats

The mean ± standard error of the serum iPTH concentrations of all individuals prior to the administration (0 hours) was 231 ± 16 pg/ml. Administrations of Compound A1 at 0.3, 3 and 30 mg/kg decreased the serum iPTH concentrations, where the means were 140, 138 and 118 mg/dL 24 hours after the administration, respectively, and 338, 280 and 245 mg/dL 48 hours after the administration, respectively. Here, the plasma Compound A1 concentration 48 hours after a single intravenous administration of Compound A1 at 30 mg/kg in the bilaterally nephrectomized rats was estimated to be 18.8 μg/ml, and a concentration sufficiently higher than the 50% effective concentration, i.e., $EC_{50}$ value, of 75 ng/ml for decreasing the serum iPTH concentration was maintained.

Meanwhile, administrations of the comparative compound at 0.3, 1, 3 and 10 mg/kg also decreased the serum iPTH concentration, where the mean serum iPTH concentrations were 122, 52, 22 and 104 mg/dL 24 hours after the administration, respectively, and 510, 280, 41 and 230 (n = 1) mg/dL 48 hours after the administration, respectively. Here, the concentration of the comparative compound in the plasma 48 hours after a single intravenous administration of the comparative compound at 3 mg/kg in the bilaterally nephrectomized rats was estimated to be 0.13 μg/ml, and a concentration sufficiently higher than the 50% effective concentration, i.e., ECso value, of 40 ng/ml for decreasing the serum iPTH concentration was maintained. Logistic curve fitting was conducted to establish the response relationship between the serum iPTH concentration 48 hours after the administration and the given dose. As a result, the lower limit values of the serum iPTH concentrations by administrations of Compound A1 and the comparative compound were simulated to be 203.0 pg/ml and 48.18 pg/ml, respectively (Fig. 2-1).

(2) Serum Ca concentration in bilaterally nephrectomized rats

The mean ± standard error of the serum Ca concentration of all individuals prior to the administration (0 hours) was 11.25 ± 0.28 mg/dL.

Administrations of Compound A1 at 0.3, 3 and 30 mg/kg decreased the serum Ca concentrations, where the means were 9.07, 7.97 and 7.99 mg/dL 24 hours after the administration, respectively, and 10.24, 8.55 and 8.14 mg/dL 48 hours after the administration, respectively.

Meanwhile, administrations of the comparative compound at 0.3, 1, 3 and 10 mg/kg also decreased the serum Ca concentration, where the mean serum Ca concentrations were 8.33, 6.42, 6.82 and 6.95 mg/dL 24 hours after the administration, respectively, and 10.44, 7.33, 5.85 and 6.65 (n = 1) mg/dL 48 hours after the administration, respectively.

Logistic curve fitting was conducted to establish the response relationship between the serum Ca concentration 48 hours after the administration and the given dose. As a result, the lower limit values of the serum Ca concentrations by administrations of Compound A1 and the comparative compound were simulated to be 8.072 mg/dL and 5.880 mg/dL, respectively (Fig. 2-2).

(3) Survival of bilaterally nephrectomized rats after single doses of Compound A1 and comparative compound

The number of rats in each group was five prior to the administration. While all five rats survived 48 hours after the administration in the medium-administered group and the Compound A1-administered groups, four survived in the 3 mg/kg group and one survived in the 10 mg/kg group among the comparative compound-administered groups (Fig. 2-3).

Compound A1 is a CaSR activator used for intravenous administration. Non-clinical studies show that renal excretion is the main route of excretion, and when the compound is intravenously administered to a normal rat, the compound is rapidly eliminated from the plasma. In renal failure rat models, serum iPTH and Ca concentrations sufficiently decreased during the 48 hours after the administration but the decreases were gradual with respect to the increase in the dose.

On the other hand, already launched etelcalcetide having the same action mechanism decreased the serum iPTH and Ca concentrations in a dose-dependent manner, but the number of dead rats increased in a dose-dependent manner.

These results show that Compound A1, as compared to etelcalcetide, had advantageous effect of not decreasing the serum iPTH and Ca concentrations to an extent affecting maintenance of life, and that it was useful for controlling the serum iPTH and Ca concentrations in nephropathy patients.

Reference Example 3: Studies of emetogenicity of Compound A1 in dogs

A crossover trial was conducted with male beagles (Nosan Beagle, 14 to 87-month-old, weight: 10.2 kg to 15.8 kg). The Compound A1 solutions used in Reference Example 2 were given at 0.3, 1 and 10 mg/kg by bolus administration (0.5 mL/kg, 1 mL/sec) to 3 to 6 dogs per dose to confirm the manifestation of vomiting immediately after the administration. Administration was conducted once or twice a week with an interval of 2 days or longer. Administration was conducted prior to feeding.
Symptoms prior to, immediately after, and 15 and 30 minutes after the administration were observed.

(Results)

The results are shown in Table 16-1.

[Table 16-1]

Table 16-1 Status of vomiting

| | Route of administration | Dose (mg/kg) | Number of vomiting cases/ Number of administration |
|---|---|---|---|
| Compound A1 | Intravenous | 0.3 | 0/3 |
| | | 1 | 0/3 |
| | | 10 | 1/6 |

For administration at 1 mg/kg with no vomiting case in the dogs, Co was 18.8 μM. Assuming that an effective dose in human was 0.1 mg/man in view of Reference Example 5 below, Co was 0.0436 μM based on the analysis results acquired by simultaneously fitting all doses.
From the above results, Compound A1 was found to have a 430 times or more higher therapeutic margin in human than in dogs with respect to vomiting.
Cinacalcet is known to frequently manifest digestive symptoms such as nausea and vomiting, which are factors that inhibit continuous administration. On the other hand, since the dose of Compound A1 of the present invention given to human greatly differs from the dose that manifests vomiting in dogs, it appears to be a medicinal composition that has few side effects such as vomiting in human, that is safe and that is adaptable to long-term administration.
Reference Example 4: Histamine release test using rat peritoneal mast cells
SD male rats (10-week-old) were used to isolate rat peritoneal mast cells according to the method of Kimura et al. (Kimura T., Eur J Pharmacol. 2000 Nov 3; 407(3): 327-32). Compound A1 was added to these mast cells to determine the amount of released histamine according to the method of Liu J et al. (Liu J J Chromatogr B Analyt Technol Biomed Life Sci. 2014 Nov 15; 971: 35-42) to compare the effects of Compound A1, the comparative compound, and Compound 48/80 as positive control on histamine release. Specifically, the test substance was added to the peritoneal cell suspension obtained from SD rats (cell concentration of $0.4 \times 10^5$ cells/ml), and determined the histamine concentration in the cell supernatant after incubated at 37°C for 30 minutes. Here, etelcalcetide was used as the comparative compound; Compound A1 and the comparative compound were each prepared into five groups of 0.1 μM to 1,000 μM in the same manner as Reference Example 2; and Compound 48/80 (manufactured by Sigma) was tested at concentrations of 0.1 mg/ml and 10.0 mg/ml.
The inhibition ratio was calculated by the following equation.

$$\text{Inhibition ratio (\%)} = (\text{Histamine concentration of individual group } (\mu M) - \text{Histamine concentration of negative control group } (\mu M)) \times 100 / (\text{Total histamine concentration } (\mu M) - \text{Histamine concentration of negative control group } (\mu M))$$

(Results)

As can be appreciated from Fig. 2-4, Compound A1 was shown to induce almost no histamine release. Meanwhile, the comparative compound increased the released histamine level in a dose-dependent manner.
Etelcalcetide is known to have the risk of developing hypersensitivity reaction, and thus requires great caution

upon administration. On the other hand, since Compound A1 of the present invention hardly causes histamine release which is a major cause of hypersensitivity reaction, it has a low probability of developing hypersensitivity reaction and thus was confirmed to be a medicinal composition with reduced side effects.

Reference Example 5: Phase I clinical trial and determination of clinically effective amount

(1) Phase I clinical trial (PI)

Thirty-two healthy Japanese male adults were given a single intravenous dose of the test drug (Compound A1) at 0.01 mg, 0.1 mg, 1.0 mg or 2.5 mg in a fasted state to study the pharmacokinetics, pharmacodynamics and safety by a double-blind test using a placebo as a control. Here, the trial drug was administered by diluting a required dose taken from the Compound A1 vial formulation prepared as follows with sterilized water for injection, and filling a syringe with the resultant in accordance with the dose to be administered.

(Compound A1 vial formulation)

A vial formulation encapsulating 100 mg of Compound A1 in terms of a dehydrate, and sodium chloride, disodium hydrogen phosphate dodecahydrate, and sodium dihydrogen phosphate dihydrate as additives in 10 ml of sterilized water for injection

(Here, the present invention can also include an aspect excluding the Compound A1 vial formulation specifically described in Reference Example 5.)

(Placebo)

A vial formulation encapsulating 10 ml of sterilized water for injection without Compound A1

(Results)

In the phase I trial that targeted healthy male adults and that used a placebo as a control, single intravenous doses of 0.01 mg, 0.1 mg, 1.0 mg and 2.5 mg of Compound A1 were present as generally unchanged substances in the plasma and rapidly eliminated. Furthermore, since they were excreted into the urine mostly as unchanged substances with respect to the given dose, renal excretion was found to be the main elimination pathway of the unchanged substance. In the pharmacodynamic evaluations, the serum iPTH concentration was confirmed to have decreased in the 0.01 mg and higher dose groups as compared to that before the administration, where the duration of decreased serum iPTH concentration extended with the increase in the given dose. As to safety, non-severe and mild side effects such as vomiting were observed in the 1.0 mg and higher dose groups, but no other problematic event was observed.

(2) Determination of clinically effective amount

A PK/PD analysis was conducted to estimate the clinically effective dose based on the results from P1. The dose of 2.5 mg seemed to be excessive and thus was eliminated from the analysis. PK (mean plasma concentration) and PD (mean iPTH level normalized by the levels of the placebo group and the 0-hour level) data of the doses of 0.01 mg, 0.1 mg and 1 mg were used. An indirect response model incorporating rebound was used for the PK/PD analysis.

Based on the results of PK of the three given doses (0.01 mg, 0.1 mg, and 1 mg) in P1, the three given doses were simultaneously analyzed using a three-compartment model to calculate the PK parameters (Fig. 2-5 and Table 16-2).

[Table 16-2]

Table 16-2 PK parameters obtained by simultaneously fitting changes in the plasma concentrations for the three given doses in P1 using a three-compartment model

| | PK parameter (± standard error) |
|---|---|
| Vd (mL/kg) | 90.9 ± 11.4 |
| Ke ($hr^{-1}$) | 1.28 ± 0.15 |
| K12 ($hr^{-1}$) | 0.658 ± 0.136 |

(continued)

| | PK parameter (± standard error) |
|---|---|
| K21 ($hr^{-1}$) | 0.819 ± 0.049 |
| K13 ($hr^{-1}$) | 4.42 ± 1.80 |
| K31 ($hr^{-1}$) | 6.89 ± 1.33 |

Using the calculated PK parameters as an input function, PK/PD analysis was performed using an indirect response model incorporating rebound (Fig. 2-6) to calculate the $EC_{50}$ value in healthy adults (Fig. 2-7 and Table 16-3).

[Table 16-3]

Table 16-3 PD parameters by PK/PD analysis of P1 using indirect response model incorporating rebound

| | PD parameter (± standard error) |
|---|---|
| $EC_{50}$ (ng/mL) | 0.421 ± 0.167 |
| Emax (ratio) | 0.920 ± 0.027 |

ECso value in patient with renal failure was estimated based on the calculated $EC_{50}$ value in healthy adults and the results from the same analysis in normal rats and adenine-treated rat models (pathological rat models). First, ECso values were calculated in the same manner in rats, which were < 22.9 ng/mL in normal rats and 74.8 ng/mL in adenine-treated rat models. Since the difference in the ECso value between rats in normal state and pathological state is presumed to be caused by the changes in PK that are considered to greatly vary between normal state and pathological state, similar tendency is expected in human as well. Accordingly, the scaling factor of $EC_{50}$ values of the healthy individuals and the patients with renal failure was set to "> 3.3". This scaling factor was applied to human, whereby $EC_{50}$ value was estimated to be > 1.4 ng/mL in the patients with renal failure.

The result from PK fitting, and the contribution rate of renal elimination of the drug (3.2%) calculated using PK-sim (registered trademark) were used to simulate PK prediction in the patients with renal failure. This simulation for PK prediction and the previously estimated $EC_{50}$ value in the patients with renal failure (> 1.4 ng/mL) were used to estimate a given dose that allows the plasma concentration of the drug to maintain the $EC_{50}$ value for 72 hours in the patients with renal failure, which was assumed to be the estimated clinically effective dose. As a result, administration of a dose of 0.06 mg/man was estimated to maintain a concentration higher than 1.4 ng/mL for 72 hours (Fig. 2-8). The clinically effective dose was estimated to be 0.1 mg/man considering that its $EC_{50}$ value exceeds 1.4 ng/mL.

Reference Example 6: Phase I/II clinical trials

Targeting SHPT patients on maintenance hemodialysis, pharmacokinetics, pharmacodynamics and safety upon single or repeated intravenous administration of Compound A1 were examined by conducting a double-blind test using a placebo as a control.

Single administration: Based on the clinically effective dose calculated in Reference Example 5, the doses were given at 0.025 mg, 0.05 mg, 0.1 mg, 0.2 mg, 0.4 mg, 0.6 mg, and 0.8 mg (7 steps). On the day of trial drug administration, the trial drug was intravenously administered as slow as possible within 60 seconds 2 to 4 hours following the end of dialysis. Here, the trial drug was administered by diluting a required dose taken from the Compound A1 vial formulation prepared as follows with sterilized water for injection, and filling a syringe with the resultant in accordance with the dose to be administered.

Repeated administration: Based on the clinically effective dose calculated in Reference Example 5, the doses were given in 3 steps, namely, 0.05 mg, 0.1 mg, and 0.2 mg. The trial drug was (intravenously) infused from the venous side of the dialysis circuit before the end of dialysis three times a week for 22 days (total of 9 times) starting from the first day of administration of the trial drug. Here, the trial drug was administered by diluting a required dose taken from the Compound A1 vial formulation prepared as follows with sterilized water for injection, and filling a syringe with the resultant in accordance with the dose to be administered.

Each item was evaluated according to the predetermined evaluation schedule.

(Compound A1 vial formulation)

A vial formulation encapsulating 1 mg of Compound A1 in terms of a dehydrate, and sodium chloride, disodium hydrogen phosphate dodecahydrate, and sodium dihydrogen phosphate dihydrate as additives in 10 ml of sterilized water for injection

(Here, the present invention can also include an aspect excluding the vial formulation of Compound A1 specifically described in Reference Example 6.)

(Placebo)

A vial formulation encapsulating 10 ml of sterilized water for injection without Compound A1

(Results)

(1) Single administration: 44 cases (Compound A1-administered groups: 29 cases, placebo group: 15 cases)

Pharmacokinetic evaluation: Cmax and AUC of Compound A1 in plasma increased with the increase in the given dose. t1/2 was 65.0 to 122 hours. When hemodialysis was performed 66 hours after the administration, the plasma Compound A1 concentration became lower immediately after the dialysis by 75 to 81% than that just before the dialysis.

Serum iPTH concentration: In the Compound A1-administered group, the serum iPTH concentration became lower than that just before the administration with a single administration, where the effect continued up to 66 hours after the administration (just before the dialysis). Here, the change in the serum iPTH concentration 66 hours after the administration was a percentage decrease of 27% in the 0.025 mg dose group, 48% in the 0.05 mg dose group, 44% in the 0.1 mg dose group, 57% in the 0.2 mg dose group, 78% in the 0.4 mg dose group, 69% in the 0.6 mg dose group, and 66% in the 0.8 mg dose group.

Safety: While relative vomiting and nausea were observed upon a single administration in the 0.4 mg and higher dose groups and in the 0.6 mg and higher dose groups, respectively, both were non-severe and mild events and no other clinical problem was observed.

(2) Repeated administration: 39 cases (Compound A1-administered group: 28 cases, and placebo group: 11 cases)

Pharmacokinetic evaluation: Compound A1 was present mainly as an unchanged substance in the plasma with repeated administration. Furthermore, since the trough concentration of Compound A1 was generally constant before the dialysis following the longest interval between the dialysis sessions, the trough concentration of Compound A1 in the plasma was found not to rise before the dialysis by the repeated administration. That is, it was found that the medicinal composition of the present invention was rapidly eliminated by dialysis and there was no accumulating property.

Serum iPTH concentration: In the Compound A1-administered group, the serum iPTH concentration decreased during the test period and thus Compound A1 maintained its effect with repeated administration. Here, the change in the serum iPTH concentration on Day 22 of the test (three days after the ninth Compound A1 administration) was a percentage decrease of 8% in the 0.05 mg dose group, 25% in the 0.1 mg dose group, and 36% in the 0.2 mg dose group.

Safety: Although decrease in the corrected Ca was observed in the 0.2 mg dose group and the 0.05 mg dose group with repeated administration, all of them were mild and none caused a problem significant to safety. Decrease in the corrected Ca was not observed in the 0.1 mg dose group.

From the above results, the medicinal composition of the present invention was found to be useful as a therapeutic agent for preventing or treating secondary hyperparathyroidism with reduced side effects when used in Japanese adults with a daily dose in a range of 0.025 mg to 0.8 mg.

INDUSTRIAL APPLICABILITY

The medicinal composition of the present invention can provide a therapeutic agent which has excellent stable production and is used for secondary hyperparathyroidism in maintenance dialysis patients.

## Claims

1. A medicinal composition comprising, as an active ingredient, 3-{[(2S)-2-amino-2-carboxyethyl]carbamoylamino}-5-chloro-4-methylbenzenesulfonic acid, a pharmaceutically acceptable salt thereof, or a solvate thereof, wherein the medicinal composition has a pH of 8.0 or less.

2. The medicinal composition according to claim 1, further comprising a pH adjuster and/or a buffer.

3. The medicinal composition according to claim 1 or 2, wherein a pH adjuster is one or a combination of two or more selected from the group consisting of an organic acid selected from acetic acid, citric acid, succinic acid, and tartaric acid, and salts thereof; an inorganic acid selected from hydrochloric acid and phosphoric acid, and salts thereof; and an inorganic base selected from sodium hydroxide and ammonia water.

4. The medicinal composition according to any one of claims 1 to 3, wherein a pH adjuster is one or a combination of two or more selected from phosphoric acid and salts thereof.

5. The medicinal composition according to any one of claims 1 to 4, wherein a pH adjuster is disodium hydrogen phosphate hydrate or sodium dihydrogen phosphate.

6. The medicinal composition according to any one of claims 1 to 5, wherein the medicinal composition has a pH of 5.0 to 7.5.

7. The medicinal composition according to any one of claims 1 to 6, wherein the medicinal composition has a pH of 6.0 to 7.0.

8. The medicinal composition according to any one of claims 1 to 7, further comprising a pharmaceutically acceptable tonicity agent.

9. The medicinal composition according to claim 8, wherein the tonicity agent is one or a combination of two or more selected from the group consisting of sodium chloride, D-mannitol, glycerin, concentrated glycerin, glucose, and propylene glycol.

10. The medicinal composition according to claim 9, wherein the tonicity agent is sodium chloride.

11. The medicinal composition according to any one of claims 1 to 10, wherein the active ingredient is used to be intravenously administered to an adult patient with secondary hyperparathyroidism under maintenance dialysis at the end of dialysis with a daily dose of 0.025 mg to 0.8 mg.

12. The medicinal composition according to claim 11, wherein the dose is 0.05 mg to 0.2 mg.

13. The medicinal composition according to claim 11 or 12, wherein the end of dialysis is the end of each dialysis session in a dialysis schedule of 3 to 5 sessions a week.

[Figure 1]

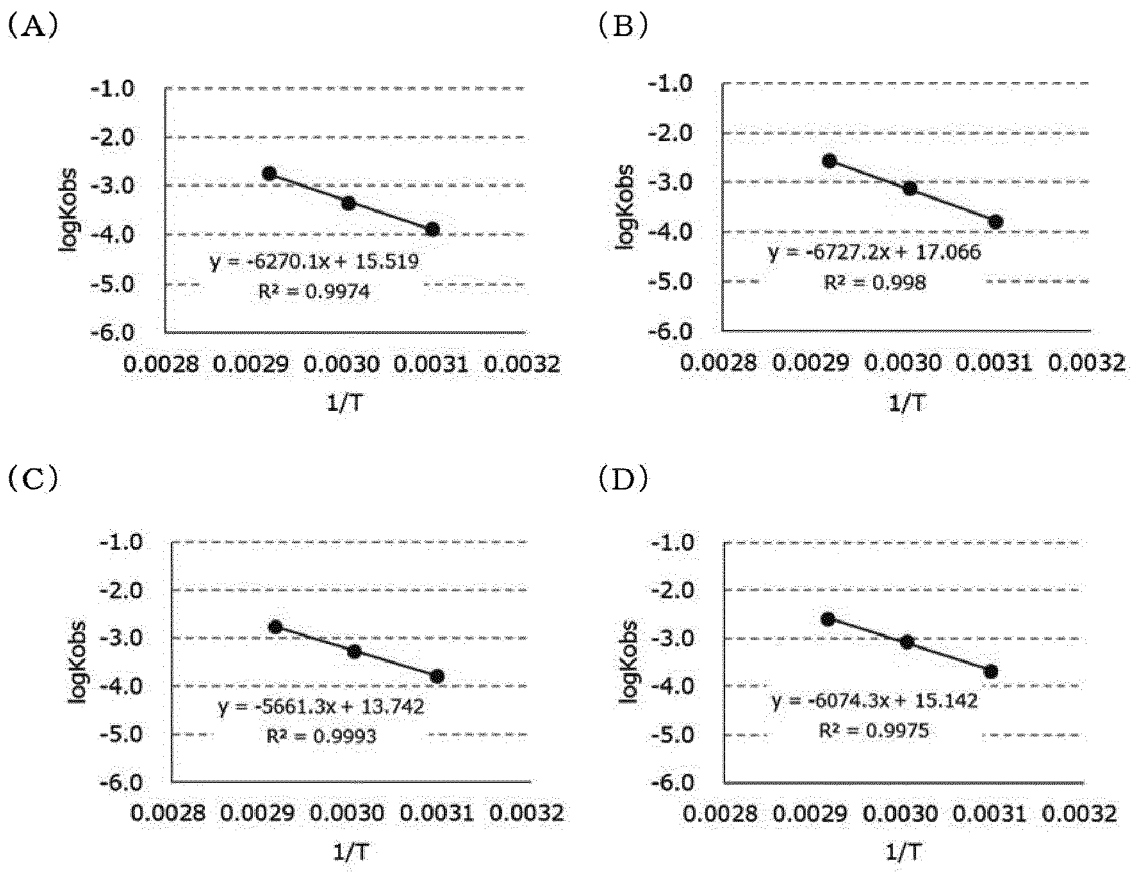


[Figure 2-1]

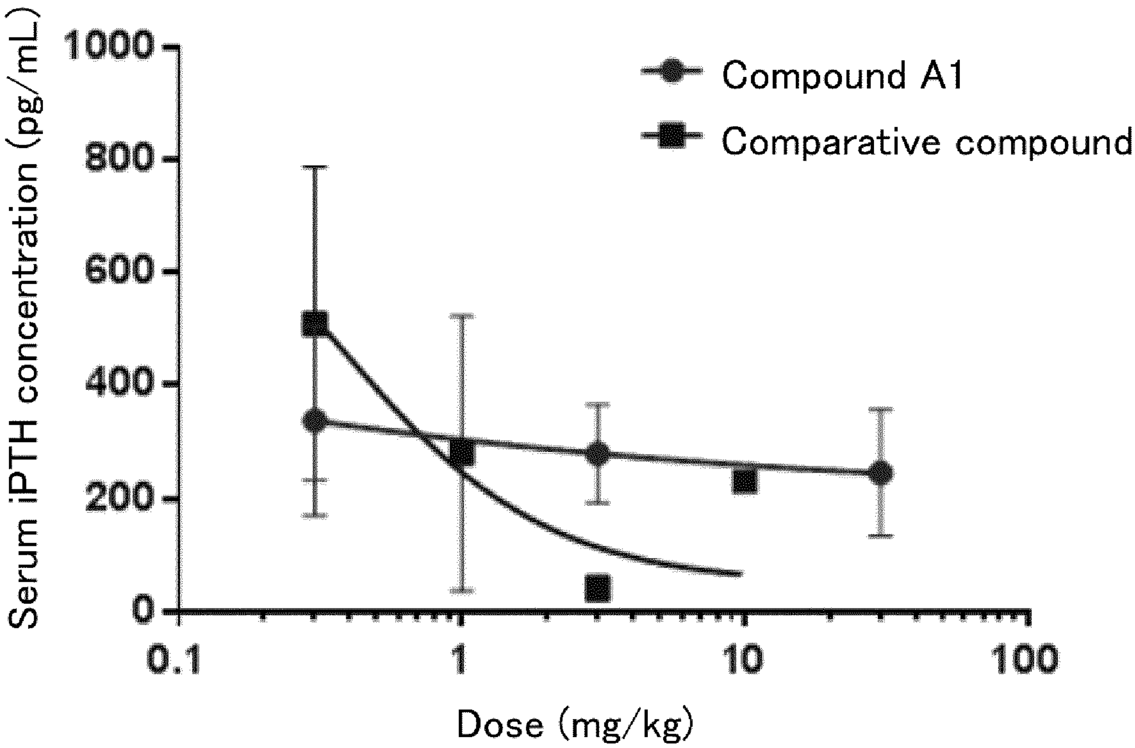


[Figure 2-2]

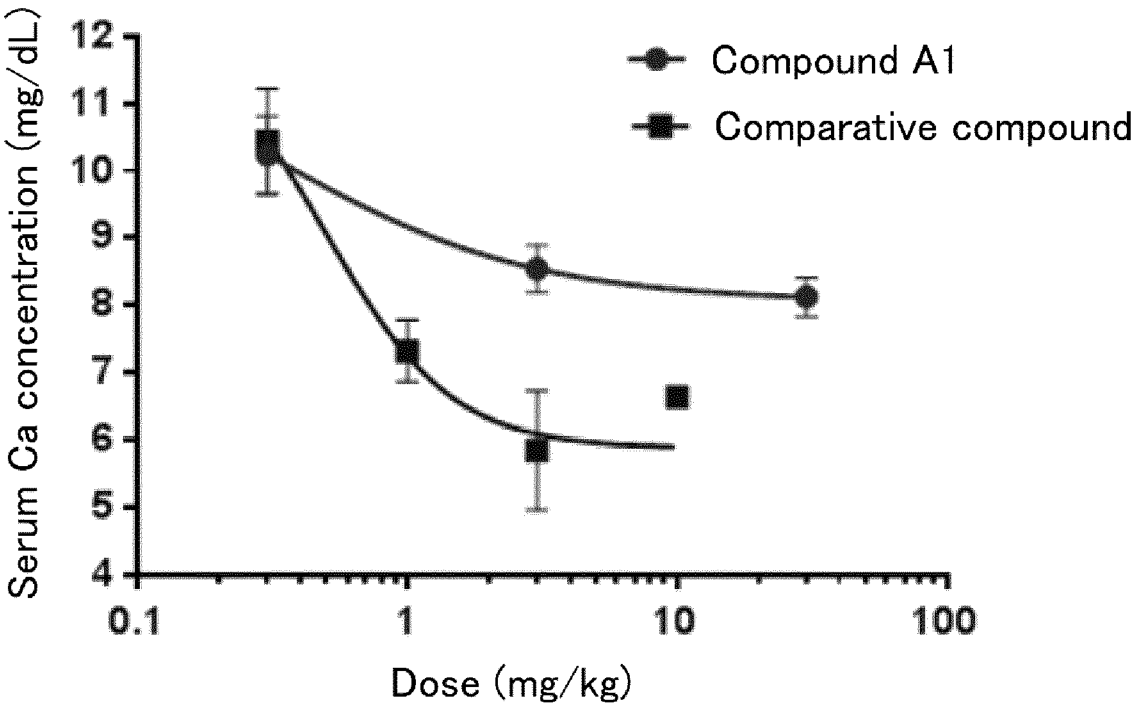


[Figure 2-3]

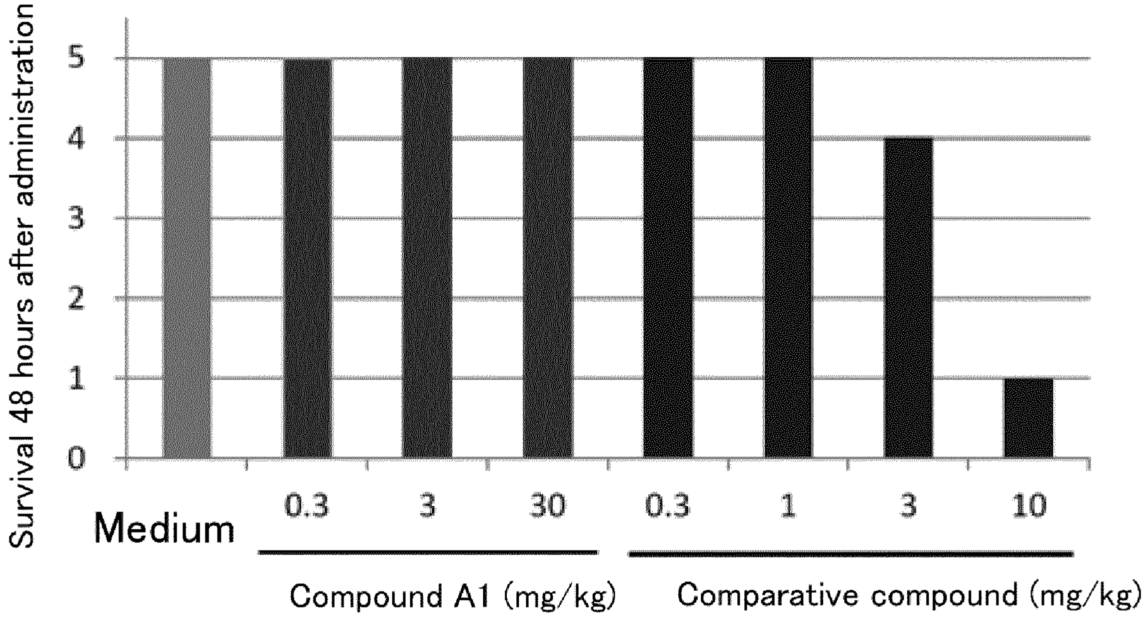
Survival 48 hours after administration
0
1
2
3
4
5
Medium
0.3
3
30
0.3
1
3
10
Compound A1 (mg/kg)
Comparative compound (mg/kg)

[Figure 2-4]

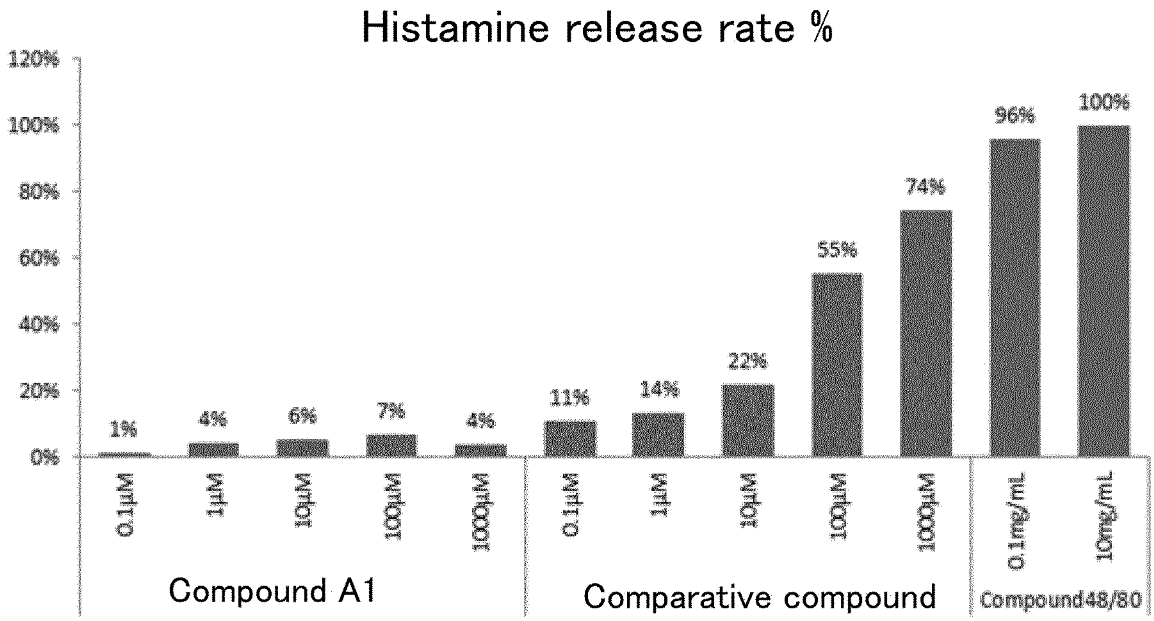
Histamine release rate %
120%
100%
80%
60%
40%
20%
0%
1%
4%
6%
7%
4%
11%
14%
22%
55%
74%
96%
100%
0.1μM
1μM
10μM
100μM
1000μM
0.1μM
1μM
10μM
100μM
1000μM
0.1mg/mL
10mg/mL
Compound A1
Comparative compound
Compound48/80

[Figure 2-5]

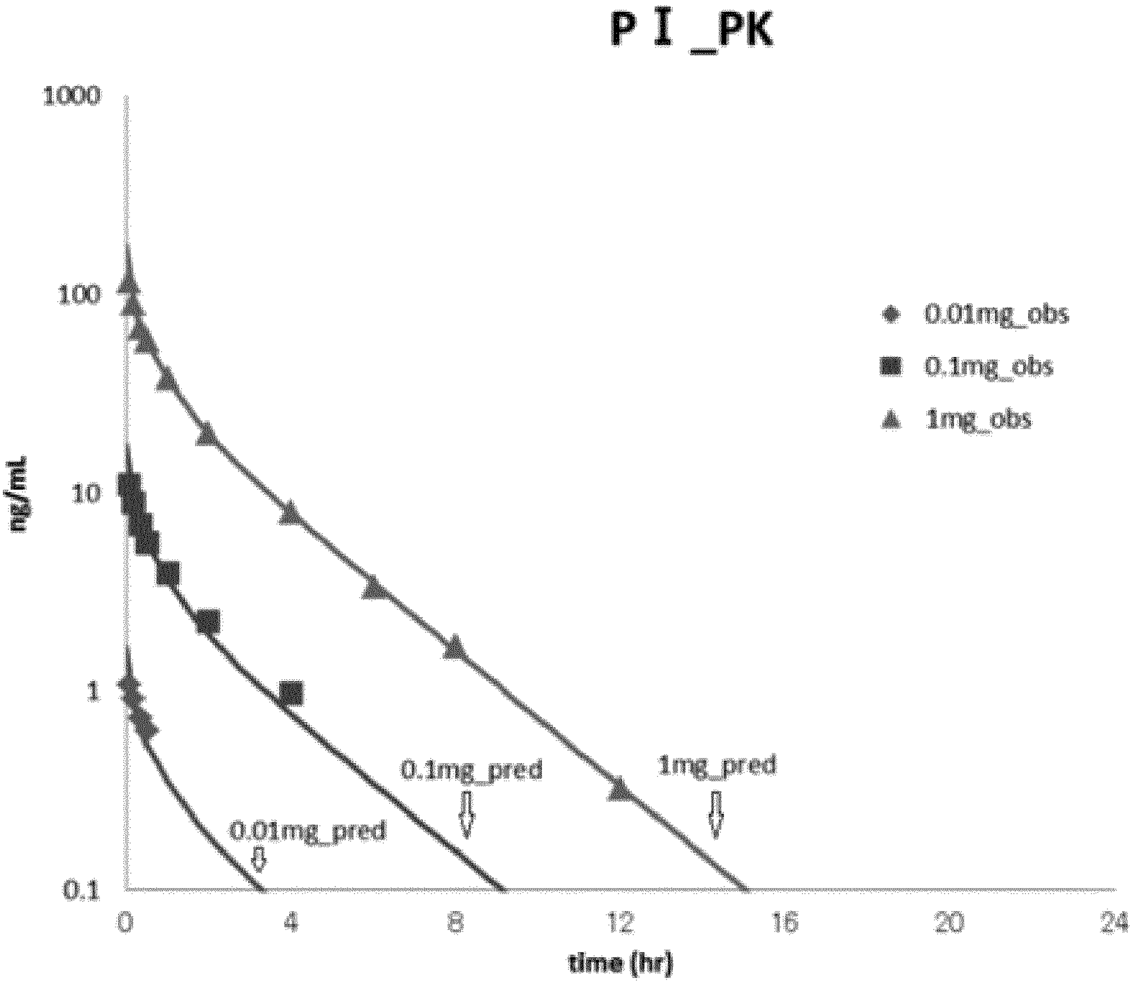
PⅠ_PK
1000
100
10
1
0.1
ng/mL
0
4
8
12
16
20
24
time (hr)
0.01mg_obs
0.1mg_obs
1mg_obs
0.01mg_pred
0.1mg_pred
1mg_pred

[Figure 2-6]

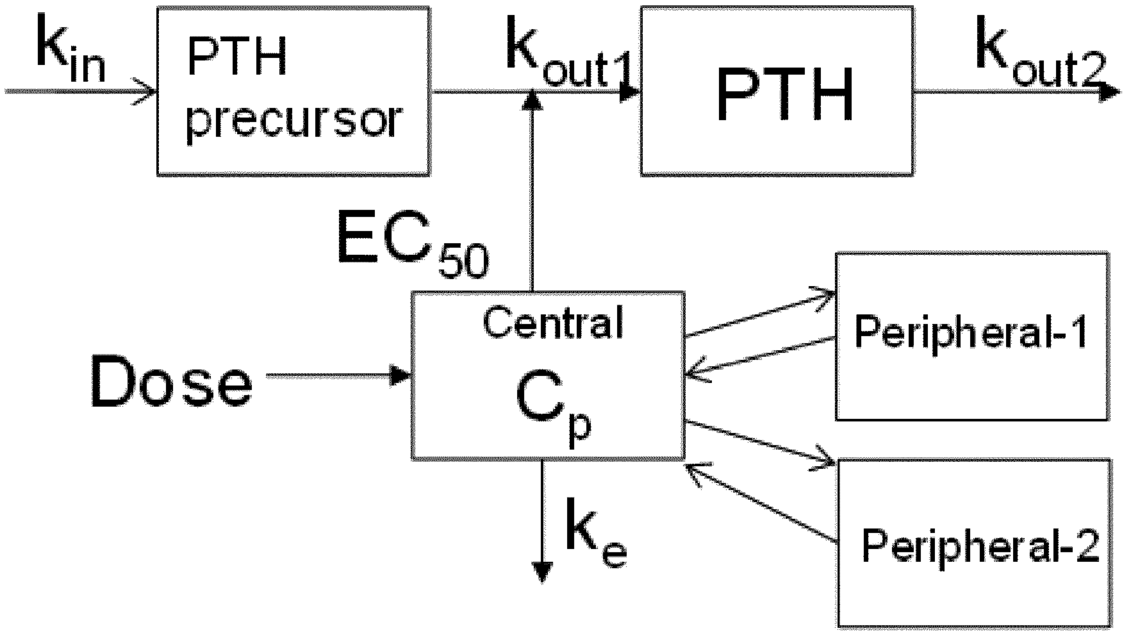
k_in
PTH precursor
k_out1
PTH
k_out2
EC_50
Dose
Central
C_p
k_e
Peripheral-1
Peripheral-2

[Figure 2-7]

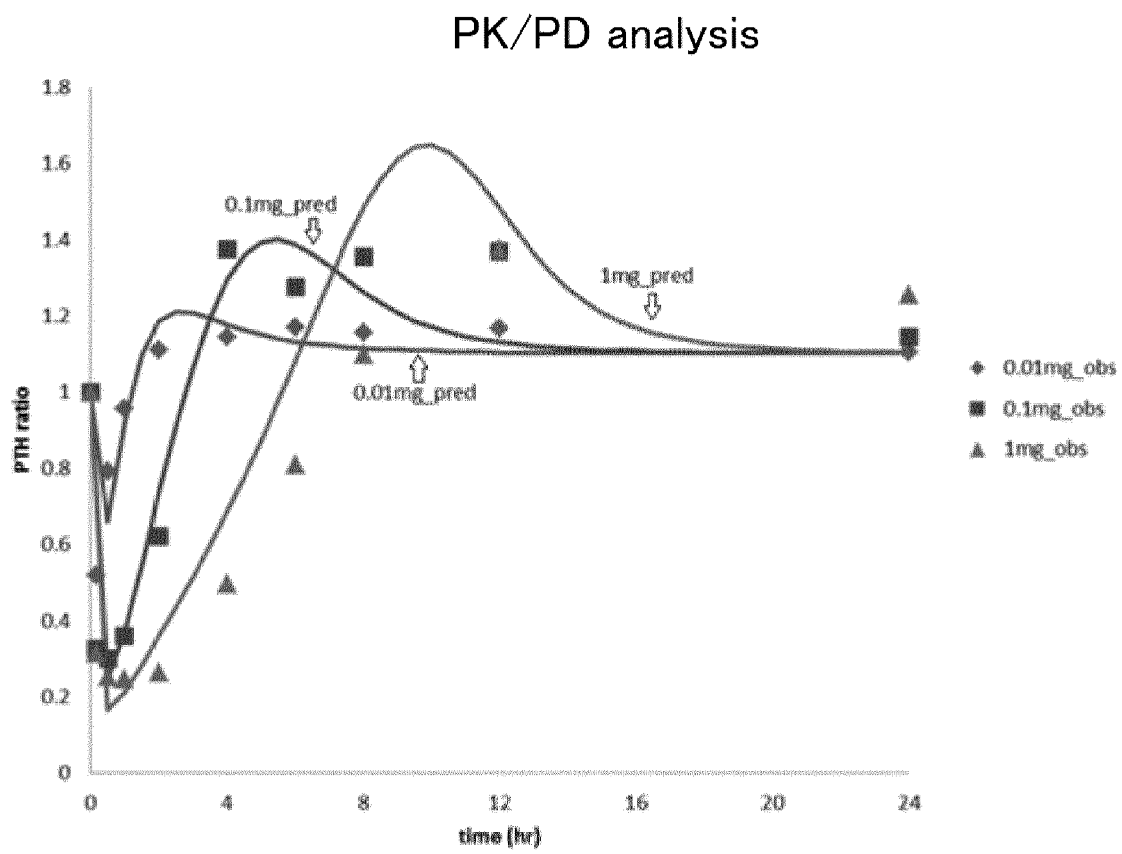
PK/PD analysis
1.8
1.6
1.4
1.2
1
0.8
0.6
0.4
0.2
0
PTH ratio
0
4
8
12
16
20
24
time (hr)
0.1mg_pred
1mg_pred
0.01mg_pred
0.01mg_obs
0.1mg_obs
1mg_obs

[Figure 2-8]

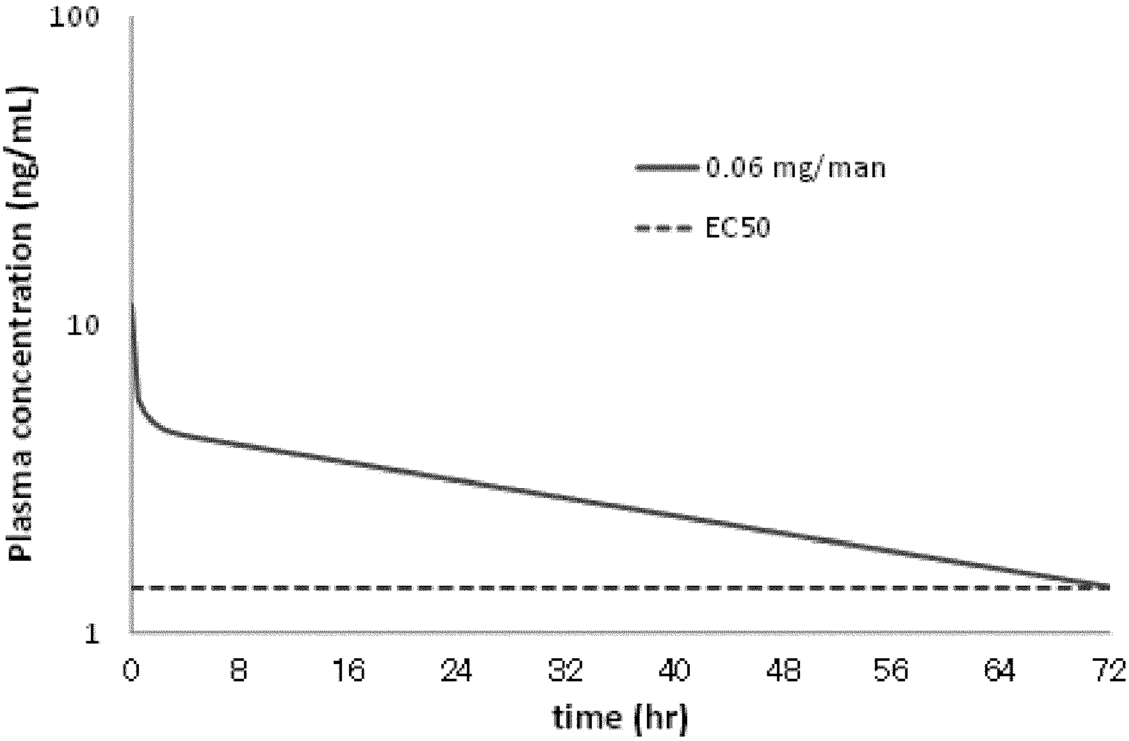
100
10
1
Plasma concentration (ng/mL)
0.06 mg/man
EC50
0
8
16
24
32
40
48
56
64
72
time (hr)

**INTERNATIONAL SEARCH REPORT**

International application No. PCT/JP2020/015129

A. CLASSIFICATION OF SUBJECT MATTER
A61P 5/18(2006.01)i; A61P 43/00 (2006.01)i; A61K 9/08(2006.01)i; A61K 47/02 (2006.01)i; A61K 47/10 (2006.01)i; A61K 47/12(2006.01)i; A61K 47/26(2006.01)i; A61K 31/198(2006.01)i
FI: A61K31/198; A61K9/08; A61K47/12; A61K47/02; A61K47/26; A61K47/10; A61P43/00 111; A61P5/18
According to International Patent Classification (IPC) or to both national classification and IPC

B. FIELDS SEARCHED
Minimum documentation searched (classification system followed by classification symbols)
A61P5/18; A61P43/00; A61K9/08; A61K47/02; A61K47/10; A61K47/12; A61K47/26; A61K31/198

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-63971 A (AJINOMOTO CO., INC.) 11.04.2013 (2013-04-11) claims 1, 3-4, paragraphs [0002], [0011], [0061], [0065], production example 14, examples I, II, III | 1-4, 6-13<br>5-13 |
| X<br>Y<br>A | WO 2011/108724 A1 (AJINOMOTO CO., INC.) 09.09.2011 (2011-09-09) claims, paragraphs [0006], [0069], [0075], example 14, test example 1 | 1-4, 6-10<br>5-10<br>11-13 |
| Y<br>A | WO 2014/119643 A1 (SENJU PHARMACEUTICAL CO., LTD.) 07.08.2014 (2014-08-07) paragraph [0041] | 5-13<br>1-4 |
| P, X | JP 2019-112396 A (EA PHARMA CO., LTD.) 11.07.2019 (2019-07-11) claims, examples | 1-13 |
| A | WO 2016/194881 A1 (AJINOMOTO CO., INC.) 08.12.2016 (2016-12-08) claims, example 3 | 1-13 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June 2020 (02.06.2020) | 16 June 2020 (16.06.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application no.
PCT/JP2020/015129

| Patent Documents referred in the Report | Publication Date | Patent Family | Publication Date |
|---|---|---|---|
| JP 2013-63971 A | 11 Apr. 2013 | (Family: none) | |
| WO 2011/108724 A1 | 09 Sep. 2011 | US 2013/0072491 A1 claims, paragraphs [0010], [0232], [0239], example 14, test example 1<br>EP 2543660 A1 | |
| WO 2014/119643 A1 | 07 Aug. 2014 | US 2015/0366969 A1 paragraph [0034]<br>EP 2952189 A1 | |
| JP 2019-112396 A | 11 Jul. 2019 | WO 2019/124411 A claims, examples | |
| WO 2016/194881 A1 | 08 Dec. 2016 | US 2018/0079715 A1 claims, example 3 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011108690 A
- JP 2013063971 A
- WO 2011014707 A

### Non-patent literature cited in the description

- **KIMURA T.** *Eur J Pharmacol.,* 03 November 2000, vol. 407 (3), 327-32
- **LIU J.** *J Chromatogr B Analyt Technol Biomed Life Sci.,* 15 November 2014, vol. 971, 35-42